# EUROPEAN PATENT APPLICATION

(11) **EP 1 426 376 A1**
(43) Date of publication of application: **09.06.2004**
(21) Application number: 02755773.5
(22) Date of filing: 02.08.2002
(51) Int. Cl.: C07D 477/06, C07D 477/08, A61K 31/407

(54) **PROCESS FOR PREPARATION OF CARBAPENEM ANTIBIOTICS**

(30) Priority: 13.08.2001 JP 2001245623
(71) Applicant: Eisai Co., Ltd., Tokyo 112-088 (JP)
(72) Inventor: TSUJII, Masahiko, Katori-gun, Chiba 289-0313 (JP); ISHIZUKA, Hiroyuki, Kashima-shi, Ibaraki 314-0006 (JP); KOIWA, Atsushi, Kashima-gun, Ibaraki 314-0343 (JP)
(74) Representative: HOFFMANN - EITLE
(86) International application number: PCT/JP2002/007891
(87) International publication number: WO 2003/016312

(57) **Abstract**

A process for the preparation of carbapenem antibiotics, which is free from the two disadvantages: (1) use of column chromatography and (2) recovery of a difficulty soluble salt and by which the objective compounds can be efficiently prepared in a short period. Specifically, a process for the preparation of compounds represented by the general formula (IV), salts thereof, or hydrates of both, characterized by reacting a compound represented by the general formula (I) with a compound represented by the general formula (II): X-H (wherein X is a member selected from the group consisting of substituents represented by the formulae (X-1) to (X-8)) to thereby obtain a compound represented by the general formula (III) or salt thereof, converting the compound (III) or salt thereof into a compound represented by the general formula (IV) or a salt thereof through the removal of the protecting group (R3), and purifying the compound (IV) or salt thereof by crystallization.

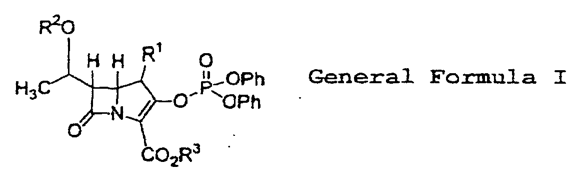

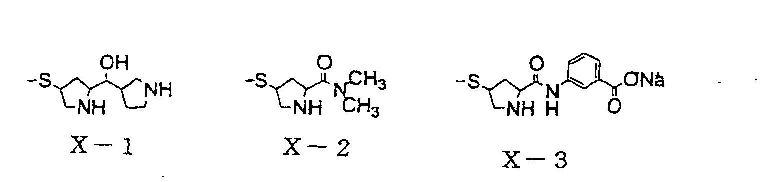

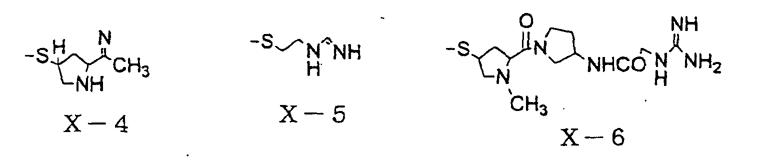

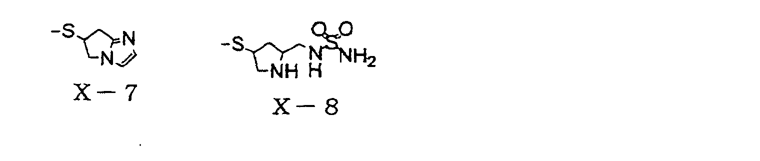

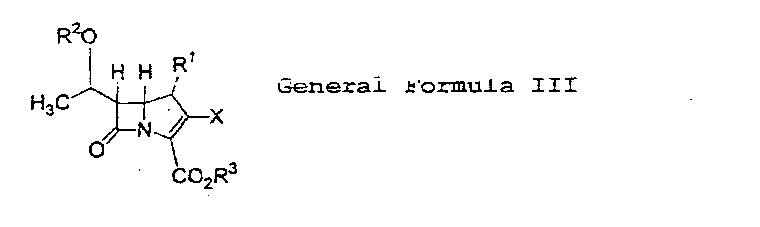

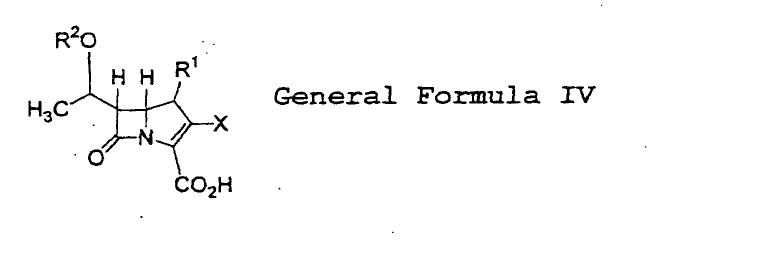

## Description

### Technical Field

The present invention relates to a process for preparing carbapenem antibiotics.

### Background Art

As to carbapenem antibiotics, many studies for synthesizing compounds have been carried out since discovery of thienamycin in 1976. In recent years, studies for carbapenem derivatives having 2-substituted pyrrolidine-4-thio group at 2-position of carbapenem skeleton have been frequently observed. Among others, the following carbapenem derivative exhibiting excellent antimicrobial action with respect to *Pseudomonas aeruginosa* is disclosed in Document 1: Drug of the Future, 1996, 21(4): 361-365.

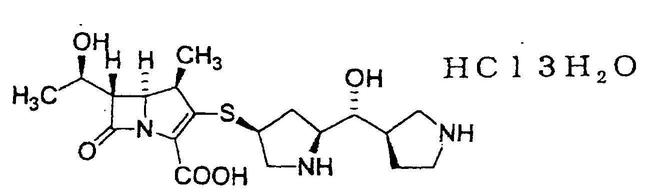

Further, the following Documents 2 to 6 have proposed a process for preparing carbapenem antibiotics (Document 2: Systematized Techniques for High Order Purification, vol. 3, Process for producing high-purity materials, 296 (1997), Fuji Techno-system; Document 3: Kohichi Hirai, Hiroshi Maruyama, Journal of Synthetic Organic Chemistry, Japan, 57, 383 (1999); Document 4: David B.R. Johnston, Susan M. Schmitt, F. Aileen BouffardandB.G. Christensen, J. Am. Chem. Soc., 100, 313 (1978); Document 5: Japanese Patent Laid-Open (JP-A) No. 11-35556; and Document 6: WO 99/45010 Official Gazette (Applicant: Merck).

The process described in Document 2 (Systematized Techniques for High Order Purification, vol. 3, Process for producing high-purity materials, 296 (1997) by Fuji Techno-system) is the one for taking once the resulting protected carbapenem out from outside the system to purify it, and then, the purified substance is freshly subjected to deprotection by means of reduction and the like and purification.

The process described in Document 3 (Kohichi Hirai, Hiroshi Maruyama, Journal of Synthetic Organic Chemistry, Japan, 57, 383 (1999)) is the one for implementing such a procedure wherein a side chain part including amine is first condensed with parent derived from azetidinone, then, an active ester is introduced into the condensate, and thereafter, the resulting product is subjected to ring-closing reaction to form a lactam ring. However, a compound into which an active ester has been already introduced, p-nitrobenzyl(1R,5S,6S)-2-diphenoxyphosphoryloxy-6-[(R)-1-hydroxyethyl]-1-methyl-1-carbapen-2-em-3-carboxylate (starting compound 1) is industrially available recently at an affordable price as a starting material for parent of carbapenem, so that the starting material has been replaced by the above-described compound. However, solubility of the starting compound 1 is.high in a high boiling point solvent such as dimethylformamide and dimethylsulfoxide, while it is low in a solvent having a low boiling point such as tetrahydrofuran and acetonitrile, as well as in aromatic solvents such as toluene (see the following Table 1). Under the circumstances, when a high boiling point solvent is used, a difficulty in industrial level arises in case of removing the high boiling point solvent as described later in detail. For this reason, there are many cases wherein the above-described starting compound 1 cannot be applied to the process of Document 3.

**Table 1.**

| Solubility of p-nitrobenzyl(1R,5S,6S)-2-diphenoxyphosphoryloxy-6-[(R)-1-hydroxyethyl)-1-methyl-1-carbapen-2-em-3-carboxylate | |
|---|---|
| Solvent | (ML/0.1g at 20 °C) |
| Tetrahydrofuran | 0.6 |
| Acetonitrile | 0.6 |
| Toluene | >10 |
| Dimethylformamide | <0.1 |
| Methylsulfoxide | <0.1 |

In the process described in Document 4 (David B. R. Johnston, Susan M. Schmitt, F. Aileen Bouffard and B. G. Christensen, J. Am. Chem. Soc., 100.313 (1978)), water/dioxane/ethanol/dipotassium hydrogen phosphate is used for a solvent in view of a problem of solubility in a case where a protected carbapenem, which had been once taken out outside the system and purified, is deprotected. Further, XAD chromatography is carried out after completing the reaction, and then a high boiling point solvent and a buffer are removed from the resulting product.

In the process described in Document 5 (JP-A No. 11-35556), a high boiling point solvent such as dimethylformamide, dimethylsulfoxide and the like is used, because of solubility of a starting material in case of synthesizing a protected carbapenem. However, since the high boiling point solvent has a high solubility, it is difficult to crystallize the resulting product, so that there has been such a disadvantage that the solvent cannot be used practically so far as it is removed through a concentration process or the like. Moreover, although the high boiling point solvent has been forcibly removed by means of a vacuum pump in an experimental laboratory, the procedure involves such a disadvantage that an expensive cost is required in view of equipment, because such procedure cannot be applied for an industrial scale unless specialized equipment is utilized.

The process described in Document 6 (WO 99/45010) uses a high boiling point solvent. However, a type of the high boiling point solvent to be used is limited to an ion pair extractable, basic solvent such as N-ethylpyrrolidone, N-methylpyrrolidone, and dimethylformamide, whereby it makes to be relatively easy the removal of the solvent.

However, any of the process disclosed in Document 1 and the processes described in Documents 2 through 6 involves a requirement for removing the high boiling point solvent. The above-described processes involve the following disadvantages from the industrial point of view, i.e., 1) column chromatography must be applied; and 2) a poor soluble salt is required to be once removed from its system.

Particularly, since the final deprotecting process is usually carried out in a more dilute aqueous solution than that of a crystallizing step, such a concentrating step wherein the aqueous solution is concentrated to several tenth percent volume of the original volume thereof is required before the crystallization, so that this is a significant problem in view of time, equipment, and stability of compound.

### Disclosure of the Invention

An object of the present invention is to solve the above-described problems.

More specifically, an object of the present invention is to provide a process for preparing carbapenem antibiotics by which two specific disadvantages, i.e., 1) column chromatography is required; and 2) a poor soluble salt must be once taken out, are eliminated, whereby the carbapenem antibiotics can be efficiently prepared for a short period of time.

Further, in the reaction applied to the present invention, a high boiling point solvent which is difficult to handle, because of difficulty in removal thereof in conventional methods, can be also used. Accordingly, the present invention also provides a process in which high solubility of such high boiling point solvents can be utilized for purification and the like.

The present inventors have carried out energetically screening of a crystallizing solvent for carbapenem antibiotics, and have found that crystallizing purification of carbapenem antibiotics becomes possible, even if a solvent such as dimethylformamide, dimethylsulfoxide and the like is contained.

Based on the fact as described above, the present inventors have found the following process: After a protected carbapenem was synthesized, the resulting product may be subjected to a deprotecting step without removing the reaction solvent. In the deprotecting step, a high boiling point solvent having excellent solubility can be also applied. In this case, since an amount of water to be used is small, no concentrating step for water is required. Further, after completing the reaction, crystallizing purification can be directly carried out, so that a cost for applying column chromatography can be reduced. As a consequence, the above-described problems can be solved, whereby carbapenem antibiotics can be prepared efficiently for a short period of time according to the present invention.

In accordance with the present invention, a target product can be taken out through a procedure wherein a protected carbapenem may be subjected to reaction in a single container up to the following deprotecting step without taking once the protected substance out and removing a solvent used, followed by just crystallizing purification (hereinafter optionally referred simply to as either "One-Pot" or "One-Pot reaction").

Moreover, when a high boiling point solvent is used, high solubility thereof can be utilized as mentioned above, and a concentrating step for water which is usually required can be omitted after completing the deprotecting reaction, whereby it is possible to effect crystallizing purification as it is.

The present invention provides the following advantages as compared with conventional processes: 1) The reaction and/or the equipment in the present invention is simple, and in addition, operability thereof is excellent, and all the steps therefor can be processed in the form of solution except for the final step. 2) Production equipments for conventional processes may be converted into those of the present invention, even when the conventional production processes are replaced with that of the present invention. 3) Purification by means of column chromatography is not required. 4) No concentrating step for water is required. 5) Required steps and the number of days for the present process become less than those of the conventional one, and e.g., the required number of days in the process of the present invention can be reduced to 1/2 to 1/3 in comparison with those of conventional processes. 6) No step for taking a product out in the middle of the reaction is required, and purification steps are less than those of conventional processes, so that a yield of the final product increases. 7) Almost all solvents each having high solubility may be used for condensation reaction, so that usability and applicability are high in the present invention. In this respect; a solvent to be used is not limited to the solvents forming ion pairs described in Document 6. 8) When a solvent having high solubility is used in a crystallizing step, it exhibits excellent effect for purifying impurities, and particularly effects for removing colored substances, organic and inorganic salts as well as for removing residual catalytic metals are observed.

More specifically, the present inventors have found that the above-described problems can be solved in accordance with the invention described hereinafter.
<1> A method for producing a compound represented by the general formula IV or the salt thereof, or the hydrates thereof, comprising the steps of:
   reacting a compound represented by the following general formula I (wherein R¹ represents a hydrogen atom or a methyl group, R² represents a hydrogen atom or a protective group for hydroxyl group, and R³ represents a hydrogen atom or a protective group for carboxyl group) with a compound represented by X-H (the general formula II) (wherein X represents anyone of substituents selected from the group consisting of the following formulae X-1 through X-8) to obtain a compound represented by the following general formula III (wherein R¹, R², R³, and X have the same meanings as defined above) or the salt thereof;
   deprotecting the protective group R³ to prepare a compound represented by the general formula IV (wherein R¹, R², and X have the same meanings as defined above) or the salt thereof; and
   crystallizing the compound represented by the general formula IV or the salt thereof thereby to purify the product: 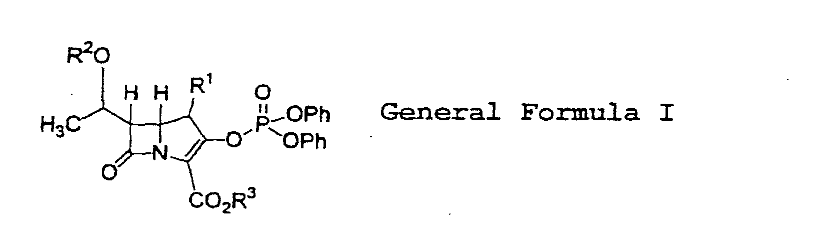 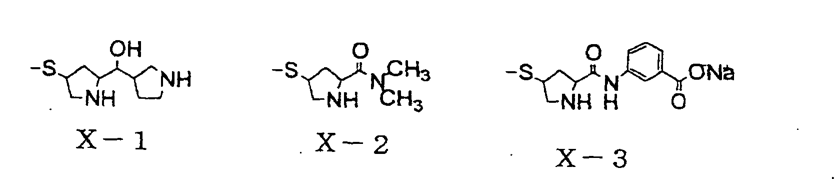 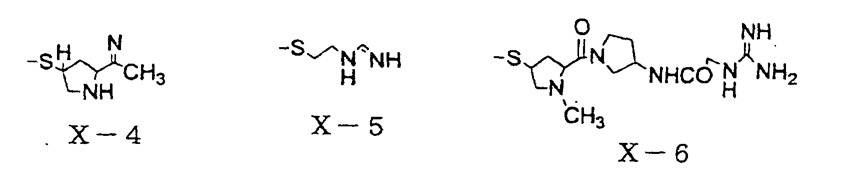 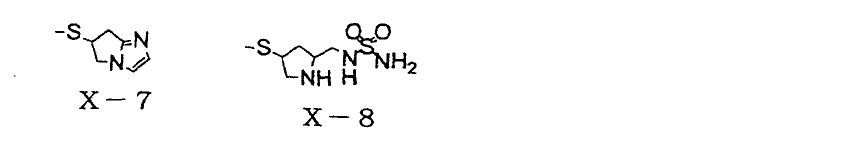 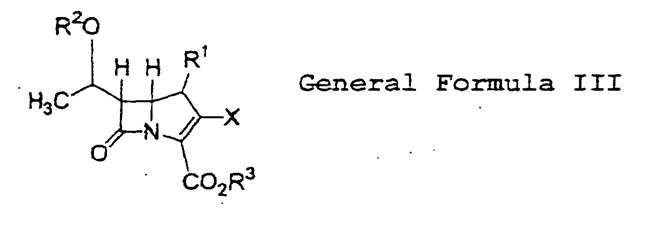 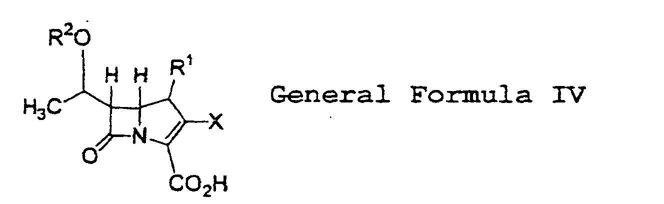
<2> A method for producing a compound represented by the general formula IV or the salt thereof, or the hydrates thereof, comprising the step of:
   crystallizing a compound represented by the following general formula IV (wherein R¹ represents a hydrogen atom or a methyl group, R² represents a hydrogen atom or a protective group for hydroxyl group, and X represents anyone of substituents selected from the group consisting of the following formulae X-1 through X-8), thereby to purify the product: 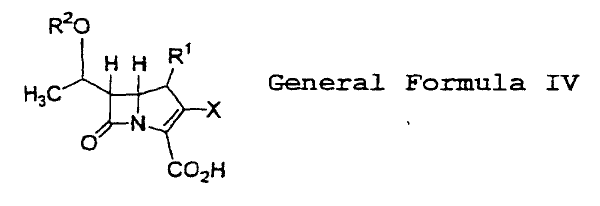 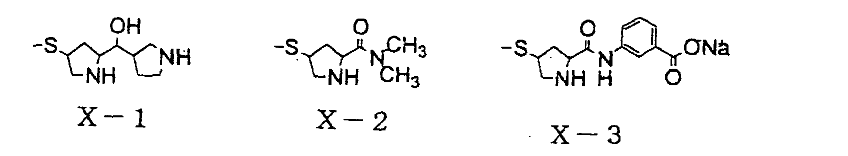 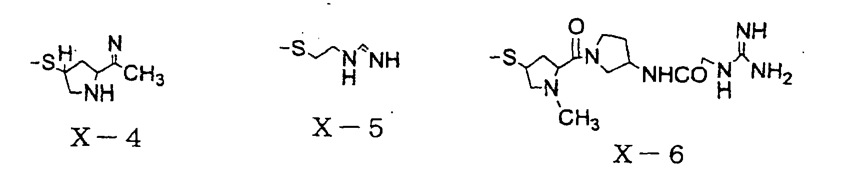 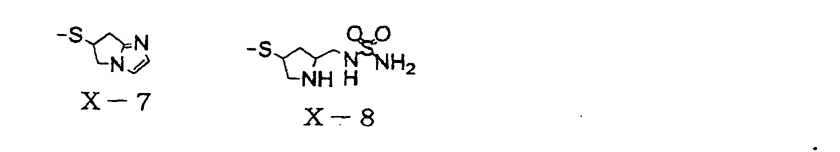
<3> . The method described in above item <1>, wherein the production is carried out in accordance with one pot manner without an isolating step.
<4> The method described in any one of above items <1> to <3>, wherein X is a substituent selected from the group consisting of the above-described formulae X-1 through X-6.
<5> The method described in any one of above items <1> to <3>, wherein X is the above-described formula X-1.
<6> The method described in any one of above items <1>, <3>, <4> and <5>, wherein said deprotecting step is carried out by catalytic reduction.
<7> The method described in any one of above items <1> to <6>, wherein said crystallizing step is carried out with the use of a solvent containing at least one member selected from the group consisting of alcohol-base solvents, dimethylsulfoxide, and dimethylformamide.
<8> The method described in above item <7>, wherein said alcohol-base solvent is at least one member selected from the group consisting of methanol, ethanol, 1-propanol, 2-propanol, 1-butanol, 2-butanol, 2-methyl-1-propanol, 2-methyl-2-propanol, 1-pentanol, 2-pentanol, and 3-pentanol.
<9> A method for producing a compound represented by the general formula IV' or the salt thereof, or the hydrates thereof, comprising the steps of:
   reacting a compound represented by the following general formula I' (wherein R¹ represents a hydrogen atom or a methyl group, R² represents a hydrogen atom or a protective group for hydroxyl group, and R³ represents a hydrogen atom or a protective group for carboxyl group) with a compound represented by X'-H (the general formula II') (wherein X' represents anyone of substituents selected from the group consisting of the following formulae X'-1 through X'-8) to obtain a compound represented by the following general formula III' (wherein R¹, R², R³, and X' have the same meanings as defined above) or the salt thereof;
   deprotecting the protective group R³ to prepare a compound represented by the general formula IV' (wherein R¹, R², and X' have the same meanings as defined above) or the salt thereof; and
   crystallizing the compound represented by the general formula IV' or the salt thereof thereby to purify the product: 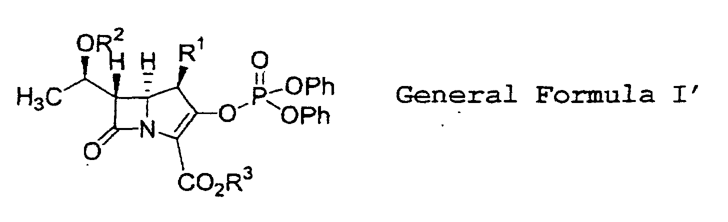 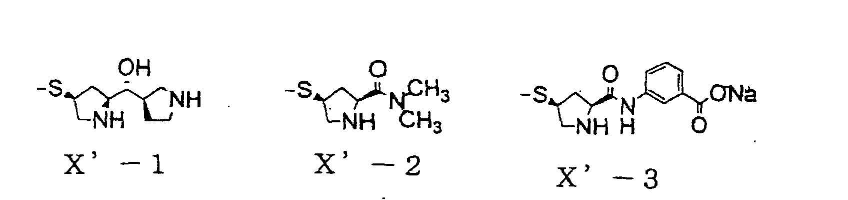 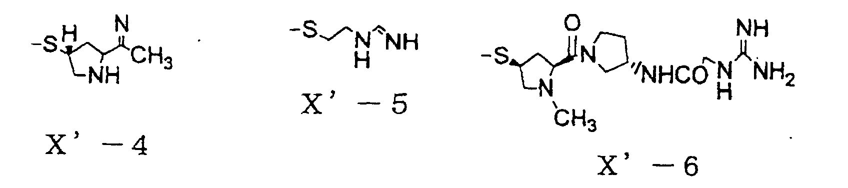 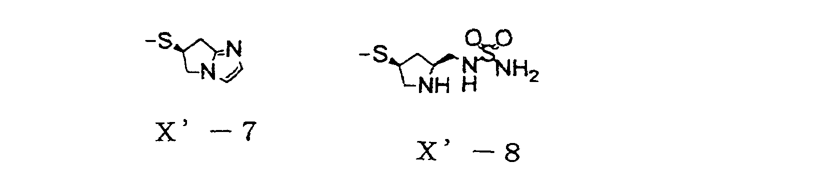 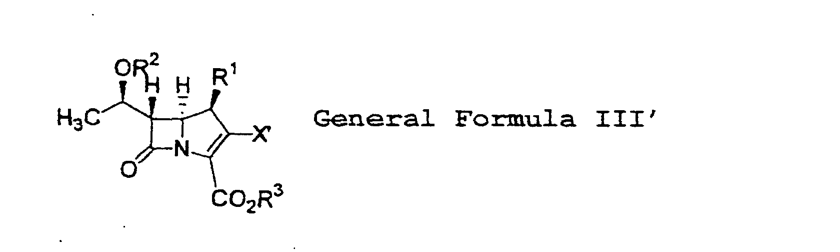 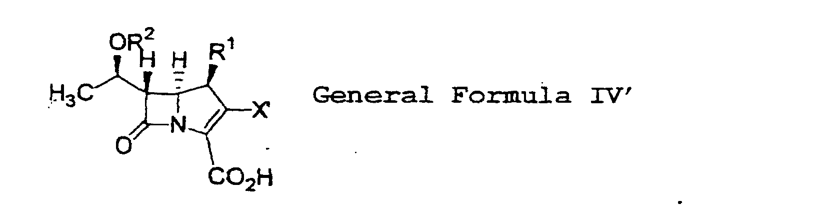
<10> A method for producing a compound represented by the general formula IV' or the salt thereof, or the hydrates thereof, comprising the step of:
   crystallizing a compound represented by the following general formula IV' (wherein R¹ represents a hydrogen atom or a methyl group, R² represents a hydrogen atom or a protective group for hydroxyl group, and X' represents anyone of substituents selected from the group consisting of the following formulae X'-1 through X'-8), thereby to purify the product: 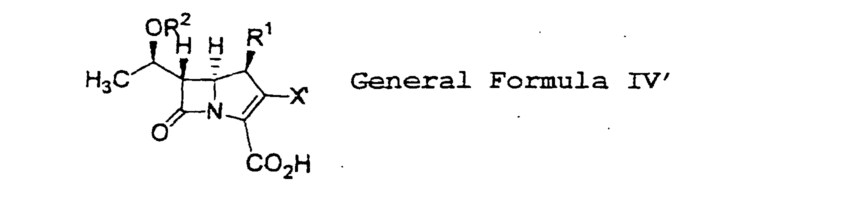 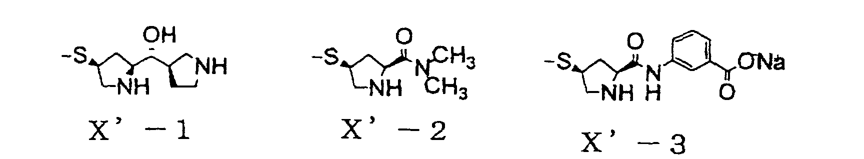 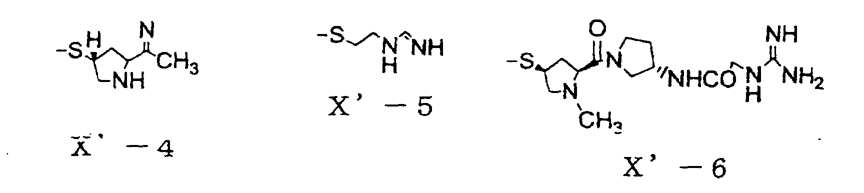 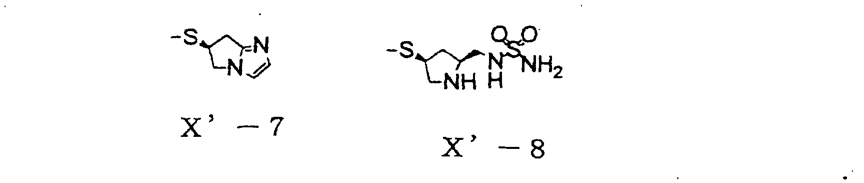
<11> The method described in above item <9>, wherein the production is carried out in accordance with one pot manner without an isolating step.
<12> The method described in any one of above items <9> to <11>, wherein X' is a substituent selected from the group consisting of the above-described formulae X'-1 through X'-6.
<13> The method described in any one of above items <9> to <11>, wherein X' is the above-described formula X'-1.
<14> The method described in any one of above items <9>, <11>, <12> and <13>, wherein said deprotecting step is carried out by catalytic reduction.
<15> The method described in any one of above items <9> to <14>, wherein said crystallizing step is carried out with the use of a solvent containing at least one member selected from the group consisting of alcohol-base solvents, dimethylsulfoxide, and dimethylformamide.
<16> The method described in above item <15>, wherein said alcohol-base solvent is at least one member selected from the group consisting of methanol, ethanol, 1-propanol, 2-propanol, 1-butanol, 2-butanol, 2-methyl-1-propanol, 2-methyl-2-propanol, 1-pentanol, 2-pentanol, and 3-pentanol.
<17> The method described in any one of above items <1>, <3> to <9>, and <10> to <16>, wherein a reaction of the compound represented by the general formula I with the compound represented by the general formula II, or a reaction of the compound represented by the general formula I' with the compound represented by the general formula II' is carried out with a use of a solvent containing dimethylsulfoxide and/or dimethylformamide.
<18> The method described in any one of above items <1>, <3> to <9>, and <10> to <17>, wherein a protective group for the compound represented by the general formula III or III' is deprotected with the use of a solvent containing dimethylsulfoxide and/or dimethylformamide to obtain the compound represented by the general formula IV or IV'.

The present invention can provide a process for preparing carbapenem antibiotics by which two specific disadvantages, i.e., 1) column chromatography is required; and 2) a poor soluble salt must be once taken out, are eliminated, whereby the carbapenem antibiotics can be efficiently prepared for a short period of time.

### Best Mode for Carrying Out the Invention

The present invention will be described in detail hereinafter.

Starting materials used in the present invention are compounds represented by the general formula I or I' and compounds represented by the general formula II or II'. These compounds may be either the ones being commercially available, or the ones which are to be newly synthesized. In case of using the synthesized ones, they may have been isolated with purification or may have not been isolated.

Compounds represented by the general formula I or I' used in the present invention will be described hereinafter.

R¹ in the compounds represented by the general formula I or I' may be a hydrogen atom or a methyl group, and preferably methyl group. R² represents a hydrogen atom or a protective group for a hydroxyl group.

The protective group for a hydroxyl group may be any group so far as it is generally known as a protective group for a hydroxyl group from the viewpoint of organic synthesis, and it is not specifically limited. Examples of such protective group for a hydroxyl group may include, but are not limited to, lower alkylsilyl groups such as trimethylsilyl group and t-butyldimethylsilyl group; lower alkoxymethyl groups such as methoxymethyl group and 2-methoxyethoxymethyl group; tetrahydropyranyl group; aralkyl groups such as benzyl group, p-methoxybenzyl group, 2,4-dimethoxybenzyl group, o-nitrobenzyl group, p-nitrobenzyl group and trityl group; acyl groups such as formyl group and acetyl group; lower alkoxycarbonyl groups such as t-buthoxycarbonyl group, 2-iodoethoxycarbonyl group and 2,2,2-trichloroethoxycarbonyl group; alkenyloxycarbonyl groups such as 2-propenyloxycarbonyl group, 2-chloro-2-propenyloxycarbonyl group, 3-methoxycarbonyl-2-propenyloxycarbonyl group, 2-methyl-2-propenyloxycarbonyl group, 2-butenyloxycarbonyl group and cinnamyloxycarbonyl group; and aralkyloxycarbonyl groups such as benzyloxycarbonyl group, p-methoxybenzyloxycarbonyl group, o-nitrobenzyloxycarbonyl group and p-nitrobenzyloxycarbonyl group.

R³ represents a hydrogen atom or a protective group for a carboxyl group. The protective group for a carboxyl group may be any group so far as it is generally known as a protective group for a carboxyl group from the viewpoint of organic synthesis, and it is not specifically limited. Examples of such protective group for a carboxyl group may include, but are not limited to, linear or branched lower alkyl groups each having 1 to 4 carbon atoms such as methyl group, ethyl group, isopropyl group and t-butyl group; halogeno lower alkyl groups such as 2-iodoethyl and 2,2,2-trichloroethyl group; lower alkoxymethyl groups such as methoxymethyl group, ethoxymethyl group and isobutoxymethyl group; lower aliphatic acyloxymethyl groups such as butyryloxymethyl group and pivaloyloxymethyl group; 1-lower alkoxycarbonyloxyethyl groups such as 1-methoxycarbonyloxyethyl group and 1-ethoxycarbonyloxyethyl group; aralkyl groups such as benzyl, p-methoxybenzyl group, o-nitrobenzyl group and p-nitrobenzyl group; benzhydryl group; and phthalidyl group.

Among others, -CH₂-Ph-NO₂ group, -CH₂-Ph group or -CH₂-CH=CH₂ group is preferable for R³.

Compounds represented by the general formula II or II' used in the present invention will be described. The compounds may be represented by either H-X or H-X' wherein X may be selected from the above-mentioned X-1 through X-8. Further, X may be preferably selected from the above-mentioned X-1 through X-6, and more preferably X may be X-1. X' may be selected from the above-mentioned X'-1 through X'-8. Further, X' may be preferably selected from the above-mentioned X'-1 through X'-6, and more preferably X' may be X'-1.

A reaction temperature in the reaction step, depending on a compound to be applied, may range generally from -40 to 50°C, preferably from -20 to 20°C.

A solvent to be used in the reaction step is not specifically limited. A solvent containing both dimethylsulfoxide and dimethylformamide, or a solvent containing either of them may also be used in the reaction step.

The present invention further involves the deprotecting step for deprotecting the protective group R³ of the compounds represented by the general formula III or III' or the salts thereof.

Any solvent may be used for a solvent to be used in the deprotecting step as long as the reaction is not adversely affected thereby and it is applied usually in organic synthesis, besides, such solvent is not specifically limited. Examples of the solvent may include, but are not limited to, lower alcohols such as methanol, ethanol, propanol, n-butanol and 3-methyl-1-butanol; polyalcohols such as ethylene glycol and glycerin; ketones such as acetone, methyl ethyl ketone, diethyl ketone and cyclohexanone; ethers such as diethyl ether, isopropyl ether, tetrahydrofuran, dioxane, 2-methoxyethanol and 1, 2-dimethoxyethane; nitriles such as acetonitrile, and propionitrile; esters such as methyl acetate, ethyl acetate, isopropyl acetate, butyl acetate and diethyl phthalate; halogenated hydrocarbons such as dichloromethane, chloroform, carbon tetrachloride, 1,2-dichloroethane, trichloroethylene and tetrachloroethylene; aromatics such as benzene, toluene, xylene, monochlorobenzene, nitrobenzene, indene, pyridine, quinoline, collidine and phenol; hydrocarbons such as pentane, cyclohexane, hexane, heptane, octane, isooctane, petroleum benzine and petroleum ether; amines such as ethanolamine, diethylamine, triethylamine, pyrrolidine, piperidine, piperazine, morpholine, aniline, dimethylaniline, benzylamine and toluidine; amides such as formamide, N-methylpyrrolidone, and N,N-dimethylformamide; amidophosphates such as hexamethyl triamidophosphate, and hexamethyl triamidophosphite; and dimethylsulfoxide, water, and the other solvents used usually, and mixed solvents prepared from two or more types thereof with a mixing ratio thereof being not specifically limited.

Examples of a solvent, which is desirably used in the deprotecting step, may include lower alcohols such as methanol, ethanol, propanol, n-butanol and 3-methyl-1-butanol; polyalcohols such as ethylene glycol and glycerin; and acetone, methyl ethyl ketone, N, N-dimethylformamide, dimethylsulfoxide, and water, and mixed solvents prepared from two or more thereof with a mixing ratio thereof is not specifically limited. Preferably, the solvent may be a lower alcohol such as methanol, ethanol, propanol, n-butanol and 3-methyl-1-butanol; and N, N-dimethylformamide, dimethylsulfoxide and water, and a mixed solvent prepared from two or more thereof with a mixing ratio thereof being not specifically limited.

More preferably the solvent may be a lower alcohol such as n-butanol and 3-methyl-1-butanol; and N,N-dimethylformamide, dimethyl sulfoxide, and water, and a mixed solvent prepared from two or more thereof with a mixing ratio thereof being not specifically limited.

A pH in the deprotecting step, depending upon the resulting compounds represented by the general formula III or III' or the salts thereof as well as upon the protective group R³ to be applied, may range from 3 to 9, preferably from 5 to 7.

A temperature in the deprotecting step, depending on the resulting compounds represented by the general formula III or III' or the salts thereof as well as on the protective group R³ to be applied as in the case of the above-mentioned pH, may range from -20 to 40°C, preferably from 4 to 30°C.

A raw material to be used in the deprotecting step may be either the one commercially available or the one which has been synthesized. In case of applying a synthesized raw material, the synthesized product may be the one which has been purified to be isolated, or the one which has not been even isolated and purified, and further, a reaction liquid in the previous condensation reaction may be applied without any modification.

As a catalyst to be applied to the deprotecting step, Pd-base, Pt-base, and Raney nickel-base catalysts may be used. From the viewpoint of reaction rate and yield, Pd-base catalysts are desirable. In this case, for example, Pd-C, Pd-black, palladium oxide, Pd/Al₂O₃, Pd/BaSO₄ and the like may be used for the Pd-base catalysts.

In the case where the protective group R³ is allyl group, a common deprotecting agent corresponding to the allyl protective group may be applied. For instance, there is a method wherein Pd(PPh₃)₄ and an acid or a salt such as ammonium formate are used in a solvent such as dioxane, tetrahydrofuran and dimethylformamide.

The process of the present invention further involves a crystallization step for crystallizing compounds represented by the general formula III or III' or the salts thereof which have been deprotected.

Examples of a solvent used for the crystallization may include solvents each containing at least an alcohol-base solvent. Examples of the alcohol-base solvents may include, but are not limited to, methanol, ethanol, 1-propanol, 2-propanol, 1-butanol, 2-butanol, 2-methyl-1-propanol, 2-methyl-2-propanol, 1-pentanol, 2-pentanol and 3-pentanol.

Water may be applied other than the above-described alcohol-base solvents as a solvent used for the crystallization, and the solvent may be a mixed solvent prepared from both water and any of these alcohol-base solvents.

When any of these solvents is used, carbapenem antibiotics can be crystallized.

Any solvent may be used for a solvent to be applied in the present invention as long as the reaction is not adversely affected thereby and it is applied usually in organic synthesis, besides, such solvent is not specifically limited. Examples of the solvent may include lower alcohols such as methanol, ethanol, propanol and butanol; polyalcohols such as ethylene glycol and glycerin; ketones such as acetone, methyl ethyl ketone, diethyl ketone and cyclohexanone; ethers such as diethyl ether, isopropyl ether, tetrahydrofuran, dioxane, 2-methoxyethanol and 1,2-dimethoxyethane; nitriles such as acetonitrile and propionitrile; esters such as methyl acetate, ethyl acetate, isopropyl acetate, butyl acetate and diethyl phthalate; halogenated hydrocarbons such as dichloromethane, chloroform, carbon tetrachloride, 1,2-dichloroethane, trichloroethylene and tetrachloroethylene; aromatics such as benzene, toluene, xylene, monochlorobenzene, nitrobenzene, indene, pyridine, quinoline, collidine and phenol; hydrocarbons such as pentane, cyclohexane, hexane, heptane, octane, isooctane, petroleum benzine and petroleum ether; amines such as ethanolamine, diethylamine, triethylamine, pyrrolidine, piperidine, piperazine, morpholine, aniline, dimethylaniline, benzylamine and toluidine; amides such as formamide, N-methylpyrrolidone and N,N-dimethylformamide; amidophosphates such as hexamethyl triamidophosphate and hexamethyl triamidophosphite; and water, and the other solvents used usually, and mixed solvents prepared from two or more types thereof with a mixing ratio thereof being not specifically limited.

As described above, any base may be used for a base to be applied in the present invention as long as the reaction is not adversely affected thereby and it is known usually as a base in organic synthesis, besides, such base is not specifically limited. Examples of the base may include sodium carbonate, sodium hydrogen carbonate, potassium carbonate, sodium hydride, potassium hydride, t-butoxy potassium, pyridine, dimethylaminopyridine, trimethylamine, triethylamine, N,N-diisopropylethylamine, N-methylmorpholine, N-methylpyrrolidine, N-methylpiperidine, N,N-dimethyl aniline, 1,8-diazabicyclo[5.4.0]undeca-7-ene (DBU), pyridine, 4-dimethylaminopyridine, picoline, lutidine, quinoline, isoquinoline, sodium hydroxide, potassium hydroxide, lithium hydroxide, butyl lithium; and sodium or potassium alcoholates such as sodium methylate, potassium methylate, and sodium ethylate.

After completing the above-mentioned reactions, the resulting product can be purified and isolated by applying a usual separating means (for example, recrystallization, chromatography and the like), if desired.

In the present specification and claims, a structural formula of a compound may conveniently represent a given isomer. Compounds of the present invention may be isomers thereof such as all geometrical isomers generated from the structure of the compounds, optical isomers based on an asymmetric carbon thereof, stereoisomers and enantiomers, and mixtures of some of the isomers. Accordingly, the compounds of the present invention are not limited to those defined by the structural formula thereof, and may be any one of the isomers, or a mixture of some of the isomers. Furthermore, the compounds of the present invention may be in a dehydrate form or in a hydrate form.

Examples of the salts referred to in the present invention may include, but are not limited to, inorganic acid additional salts such as hydrofluorides, hydrochlorides, sulfates, nitrates, perchlorates, phosphates, carbonates, bicarbonates, hydrobromates, hydroiodides and the like; organic carboxylate additional salts such as acetates, maleates, fumarates, oxalates, lactates, tartrates, trifluoroacetates and the like; organic sulfonate additional salts such as methanesulfonates, trifluoromethanesulfonates, ethanesulfonates, hydroxymethanesulfonates, hydroxyethanesulfonates, benzenesulfonates, toluenesulfonates, taurine salts and the like; amine additional salts such as trimethylamine salts, triethylamine salts, pyridine salts, procaine salts, picoline salts, dicyclohexylamine salts, N,N-dibenzylethylenediamine salts, N-methylglucamine salts, diethanolamine salts, triethanolamine salts, tris(hydroxymethylamino)methane salts, phenetylbenzylamine salts and the like; alkali metal additional salts such as sodium salts, potassium salts and the like; alkali earth metal additional salts such as magnesium salts, calcium salts and the like; and amino acid additional salts such as arginine salts, lysine salts, serine salts, glycine salts, aspartate salts, glutamate salts and the like. Preferably, pharmaceutically acceptable salts are used.

### Examples

Hereinafter, the present invention will be illustrated by means of examples, but it should be appreciated that the present invention is not limited to the examples.

### Example 1:

### <Synthesis of P-nitrobenzyl(1R,5S,6S)-2-diphenoxyphosphoryloxy-6-[P-nitro benzyl(1R,5S,6S)-2-diphenoxyphosphoryloxy-6-[(R)-hydroxyeth yl]-1-methyl-1-carbapen-2-em-3-carboxylic acid from a starting material: P-nitrobenzyl(1R,5S,6S)-2-diphenoxyphophoryloxy-6-[(R)-1-hy droxyethyl]-1-methyl-1-carbapen-2-em-3-carboxylate; condensation reaction in a dimethylsulfoxide solution>

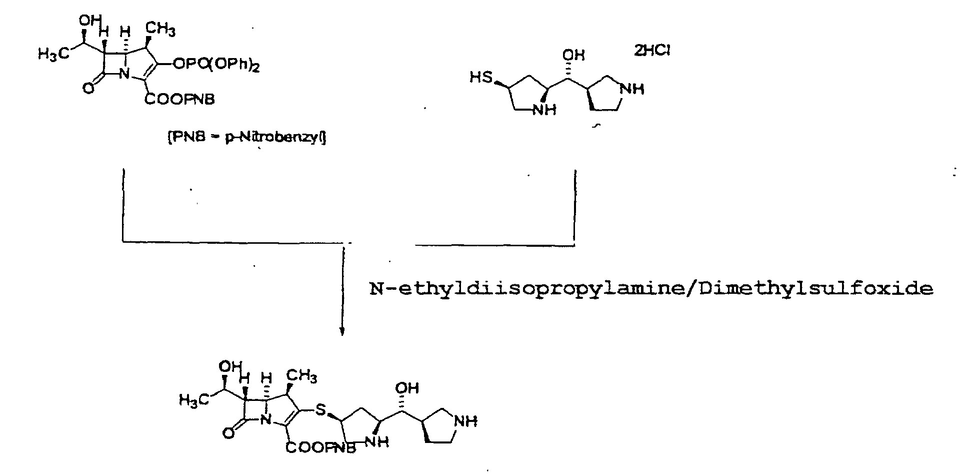

P-nitrobenzyl(1R,5S,6S)-2-diphenoxyphosphoryloxy-6-[(R)-3-hydroxyethyl]-1-methyl-1-carbapen-2-em-3-carboxylate (5.0 g, 8.41 mmol) was added into a 200 mL four-necked flask, and then dimethylsulfoxide (45 mL) was added thereto. The mixture was dissolved at room temperature (19°C), and then, N-ethyldiisopropylamine (2.88 mL, 16.8 mmol, 2.0 eq.) was added (at 18 to 19°C) with stirring. Then, (2S,4S)-2-[[(3R)-pyrrolidin-3-yl-(R)-hydroxy]methyl]-4-mercaptopyrrolidine dihydrochloride (2.55 g, 9.26 mmol, 1.1 eq.) powder was slowly added to the resulting mixture for five minutes with stirring.

The admixture was maintained at 18 to 20°C for thirty minutes, and the produced P-nitrobenzyl(1R,5S,6S)-2-diphenoxyphosphoryloxy-6-[P-nitrobenzyl(1R,5S,6S)-2-diphenoxyphosphoryloxy-6-[(R)-hydroxyethyl]-1-methyl-1-carbapen-2-em-3-carboxylic acid was quantitatively determined by means of HPLC.

HPLC determination: (quantitative) JASCO HPLC PUMP PU-980, GL Science Inc. Inertsil ODS-2 (4.6 x 150 mm), JASCO HPLC UV/VIS UV-970 DETECTOR; wavelength 295 nm, rate of flow 1.5 mL/min.; developing solvent: acetonitrile/H₂O/phospholic acid/dodecyl sodium sulfate = 500 mL/450 mL/1 mL/1.44 g.

### Example 2:

### <Synthesis of P-nitrobenzyl(1R,5S,6S)-2-diphenoxyphosphoryloxy-6-[(R)-hydroxyethyl]-1-methyl-1-carbapen-2-em-3-carbixylic acid from P-nitrobenzyl(1R,5S,6S)-2-diphenoxyphosphoryloxy-6-[(R)-1-hydroxyethyl]-1-methyl-1-carbapen-2-em-3-carboxylate ; condensation reaction in dimethylsulfoxide/dimethylformamide = 2/1 mixed solvent>

P-nitrobenzyl(1R,5S,6S)-2-diphenoxyphosphoryloxy-6-[(R)-1-hydroxyethyl]-1-methyl-1-carbapen-2-em-3-carboxylate (5.0 g, 8.41 mmol) was added into a 200 mL four-necked flask, and then a mixture of dimethylsulfoxide (30 mL) and dimethylformamide (5 mL) was added thereto. The resulting mixture was dissolved at room temperature (18°C), and then, N-ethyldiisopropylamine (2. 88 mL, 16. 8 mmol, 2.0 eq.) was added (at 16 to 17°C) with stirring. Then, (2S,4S)-2-[[(3R)-pyrrolidin-3-yl-(R)-hydroxy]methyl]-4-mercaptopyrrolidine dihydrochloride (2.55 g, 9.26 mmol, 1.1 eq.) powder was slowly added to the resulting mixture for five minutes with stirring.

The admixture was maintained at 10 to 14°C for thirty minutes, and the produced P-nitrobenzyl(1R,5S,6S)-2-diphenoxyphosphoryloxy-6-[P-nitrobenzyl(1R,5S,6S)-2-diphenoxyphosphoryloxy-6-[(R)-hydroxyethyl]-1-methyl-1-carbapen-2-em-3-carboxylic acid was quantitatively determined by means of HPLC, in a manner similar to Example 1.

### Example 3:

### <One-Pot reaction of (1R,5S,6S)-6-[(R)-1-hydroxyethyl]-1-methyl-2-{(2S,4S)-2-[(3R)-pyrrolidin-3-yl-(R)-hydroxy]methyl}pyrrolidine-4-ylthio]-1-carbapen-2-em-3-carboxylic acid monohydrochloride trihydrate from P-nitrobenzyl(1R,5S,6S)-2-diphenoxyphophoryloxy-6-[(R)-hydroxyethyl]-1-methyl-1-carbapen-2-em-3-carboxylic acid, which was derived from a starting material: P-nitrobenzyl(1R,5S,6S)-2-diphenoxyphosphoryloxy-6-[(R)-1-hydroxyethyl]-1-methyl-1-carbapen-2-em-3-carboxylate ; reduction by means of 5% Pd-C under a condition of two phase reaction 1-butanol/H₂O>

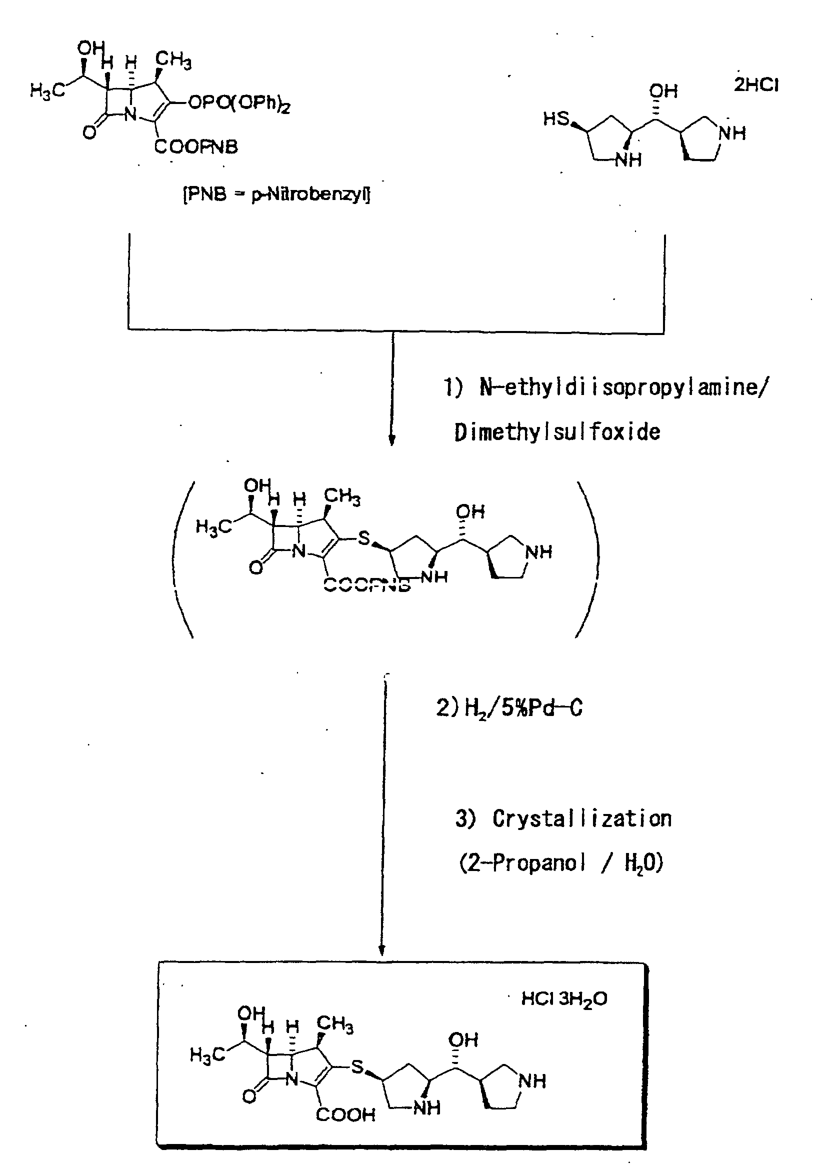

P-nitrobenzyl(1R,5S,6S)-2-diphenoxyphosphoryloxy-6-[(R)-1-hydroxyethyl]-1-methyl-1-carbapen-2-em-3-carboxylate (5.0 g, 8.41 mmol) was added into a 200 mL four-necked flask, and then, dimethylsulfoxide .(45 mL) was added thereto. The mixture was dissolved at room temperature (19°C), and then, N-ethyldiisopropylamine (3. 66 mL, 21.3 mmol, 2.6 eq.) was added thereto (at 17 to 18°C) with stirring. Then, (2S,4S)-2-[[(3R)-pyrrolidin-3-yl-(R)-hydroxy]methyl]-4-mercaptopyrrolidine dihydrochloride (2.55 g, 9.26 mmol, 1.1 eq.) powder was slowly added to the resulting mixture for five minutes with stirring. The admixture was maintained at 17 to 18°C for thirty minutes thereby allowing the same to react with each other.

The reaction solution was diluted with 1-butanol (60 mL). Separately, a mixed solution of distilled water (270 mL), 5% Pd-C (6.0 g), and 1-butanol (60 mL) was previously prepared in a 500 mL four-necked flask equipped with a pH stat and a stirrer. To the mixed solution was added the resulting reaction solution diluted with 1-butanol with stirring. 1-Butanol (60 mL) was further added to the 200 mL flask to which addition of the reaction solution had been completed to wash the flask, and the washing liquid was also added to the 500 mL flask. During the addition of the reaction solution and the washing liquid, the temperature was maintained at 10°C or less, and the pH was maintained at 4.5 to 6.5. After the addition of the reaction solution and the washing liquid, the inside of the 500 mL flask was replaced with nitrogen. Thereafter, the admixture was vigorously stirred for four hours while adjusting a pH of the reaction solution to 6. 0 by dropping 1N sodium hydroxide aqueous solution by means of a constant rate pump linked to the pH stat under hydrogen atmosphere (normal pressure, hydrogen feeding by means of a balloon).

The hydrogen feeding was stopped at the time when consumption of the 1N sodium hydroxide aqueous solution ceased (total 9.0 mL), and thereafter, a catalyst was filtered off by the use of a Kiriyama funnel (No. 5). The remaining liquid was washed with distilled water (35 mL x 2), and thereafter, the filtrate and the washing solutions were mixed. When the organic layer and the water layer were subjected to HPLC determination, respectively, it was found that a free type of the titled compound produced was as follows. (Water layer: 2.94 g; organic layer: 0.0045 g; total reaction yield from P-nitrobenzyl(1R,5S,6S)-2-diphenoxyphosphoryloxy-6-[(R)-1-hydroxyethyl]-1-methyl-1-carbapen-2-em-3-carboxylate : 85.0%)

The above-described water layer was subjected to vacuum concentration at a temperature of 20°C or less until an amount of the water layer reached 140 g. Thereafter, the water layer thus concentrated was treated with activated carbon (Taikoh FCS, 0.5 g) for 10 minutes, and then, the activated carbon was filtered off. A filtrate (125 g) combined with wash liquid (15 mL) for the filter residue was cooled to 10°C. To the resulting solution, IPA (190 mL) was added dropwise with stirring, and seed crystal (0.17g) of the titled compound was added thereto. After fifteen minutes, precipitate of crystal was confirmed, and IPA (200 mL) was added dropwise. The admixture was crystallized at 10°C for 16 hours, thereafter, the crystal was filtered out by means of suction filtration. The crystal was washed with 80% IPA aqueous solution (15 mL), IPA (15 mL), and acetone (15 mL) in this order. The resulting crystal was dried by means of suction for thirty minutes under flowing nitrogen, to obtain 3.24 g of the titled compound (corresponding to 2.64 g when converted into the free type; recovery excluding an amount of the added seed crystal: 84.1%).

After dissolving 2.01 g (corresponding to 1.64 g in the form of the free type thereof) of (1R,5S,6S)-6-[(R)-1-hydroxylethyl]-1-methyl-2-{(2S,4S)-2-[(3R)-pyrrolidin-3-yl-(R)-hydroxy]methyl}pyrrolidin-4-ylthio]-1-carbapen-2-em-3-carboxylic acid monochloride trihydrate into distilled water (20 mL), contaminant was filtered off (0.2 µm filter) from the resulting solution. 2-Propanol (69 mL) was added dropwise to an aqueous solution combined with washing aqueous solution (8 mL) for forty four minutes with stirring the mixture under cooling at 10°C. Seed crystal (0.17 g) of the titled compound was added thereto. After fifteen minutes, precipitate of crystal was confirmed, and then, the crystal was aged for one hour, thereafter, 2-propanol (150 mL) was further added dropwise thereto for two hours. After aging for one hour, the crystal deposited was filtered off, the resulting crystal was sequentially washed with 80% (v/v) 2-propanol aqueous solution (15 mL), 2-propanol (15 mL), and acetone (15 mL). The product was dried (for one hour) on the filter while depressurizing and drafting the same under nitrogen stream to obtain 2.00 g. of the target titled crystal (corresponding to 1.69 g when converted into the free type; recovery excluding an amount of the added seed crystal: 94.9%). ¹H-NMR(D₂O) : δ1.08 (3H, d, J=7Hz), 1.16 (3H, d, J=7Hz), 1.63 (1H, qd, J=9,13Hz), 1.76 (1H, ddd, J=7, 10, 13Hz), 1.98-2.07 (1H, m), 2.35 (1H, qd, J=9,17Hz), 2.50 (1H, td, J=8,14Hz), 3.06 (1H, dd, J=9,12Hz), 3.14-3.26 (3H, m), 3.32-3.37 (2H, m), 3.42(1H, dd, J=8,12Hz), 3.57 (1H, dd, J=7,12Hz), 3.78 (1H, ddd, J=3, 8, 11Hz), 3.86-3.91 (2H, m), 4.08-4.13, (2H, m).

NMR data of the resulting crystal of the titled compound were corresponding to the titled compound, which was synthesized in a separate manner.

HPLC determination: (quantitative) JASCO HPLC PUMP PU-980, GL Science Inc. Inertsil ODS-2 (4.6 x 150 mm), JASCO HPLC UV/VIS UV-970 DETECTOR; wavelength 295 nm, rate of flow 1.0 mL/min.; developing solvent: methanol/H₂O/ammonium acetate = 160 mL/1840 mL/2 g.

### Example 4:

### <One-Pot reaction of (1R,5S,6S)-6-[(R)-1-hydroxyethyl]-1-methyl-2-{(2S,4S)-2-[(3R)-pyrrolidin-3-yl-(R)-hydroxy]methyl}pyrrolidine-4-ylthio]-1-carbapen-2-em-3-carboxylic acid monohydrochloride trihydrate from P-nitrobenzyl(1R,5S,6S)-2-diphenoxyphophoryloxy-6-[(R)-hydroxyethyl]-1-methyl-1-carbapen-2-em-3-carboxylic acid, which was derived from a starting material: P-nitrobenzyl(1R,5S,6S)-2-diphenoxyphosphoryloxy-6-[(R)-1-hydroxyethyl]-1-methyl-1-carbapen-2-em-3-carboxylate ; reduction by means of 5% Pd-C under a condition of two phase reaction 3-methyl-1-butanol/H₂O>

P-nitrobenzyl(1R,5S,6S)-2-diphenoxyphosphoryloxy-6-[(R)-1-hydroxyethyl]-1-methyl-1-carbapen-2-em-3-carboxylate (5.0 g, 8.41 mmol) was added into a 200 mL four-necked flask, and then, dimethylsulfoxide (45 mL) was added thereto. The mixture was dissolved at room temperature (19°C), and then, N-ethyldiisopropylamine (3.66mL, 21.3 mmol, 2.6 eq.) was added thereto (at 16 to 19°C) with stirring. Then, (2S,4S)-2-[[(3R)-pyrrolidin-3-yl-(R)-hydroxy]methyl]-4-mercaptopyrrolidine dihydrochloride (2.55 g, 9.26 mmol, 1.1 eq.) powder was slowly added to the resulting mixture for five minutes with stirring. The admixture was maintained at 17 to 18°C for thirty minutes thereby allowing the same to react with each other.

The reaction solution was diluted with 3-methyl-1-butanol (60 mL). Separately, a mixed solution of distilled water (135 mL), 5% Pd-C (6.0 g), and 3-methyl-1-butanol (60 mL) was previously prepared in a 500 mL four-necked flask equipped with a pH stat and a stirrer. To the mixed solution was added the resulting reaction solution with stirring. 3-Methyl-1-butanol (60 mL) was further added to the 200 mL flask to which addition of the reaction solution had been completed to wash the flask, and the washing liquids were also added to the 500 mL flask. During the addition of the reaction solution and the washing liquids, the temperature was maintained at 10°C or less, and the pH was maintained at 4.5 to 6.5. After the addition of the reaction solution and the washing liquids, the inside of the 500 mL flask was replaced with nitrogen. Thereafter, the admixture was vigorously stirred for four hours while adjusting a pH of the reaction solution to 6.0 by dropping 1N sodium hydroxide aqueous solution by means of a constant rate pump linked to the pH stat under hydrogen atmosphere (normal pressure, hydrogen feeding by means of a balloon).

The hydrogen feeding was stopped at the time when consumption of the 1N sodium hydroxide aqueous solution ceased (total 9.7 mL), and thereafter, a catalyst was filtered off by the use of a Kiriyama funnel (No. 5). The remaining liquid was washed with distilled water (35 mL x 2), and thereafter, the filtrate and the washing solutions were combined. When the organic layer and the water layer were subjected to HPLC determination, respectively, it was found that a free type of the titled compound produced was as follows. (Water layer: 2.91 g; organic layer: 0.0096 g; total reaction yield from P-nitrobenzyl(1R,5S,6S)-2-diphenoxyphosphoryloxy-6-[(R)-1-hydroxyethyl]-1-methyl-1-carbapen-2-em-3-carboxylate : 84.3%)

### Example 5:

### <One-Pot reaction of (1R,5S,6S)-6-[(-R)-1-hydroxyethyl]-1-methyl-2-{(2S,4S)-2-[(3R)-pyrrolidin-3-yl-(R)-hydroxy]methyl}pyrrolidine-4-ylthio]-1-carbapen-2-em-3-carboxylic acid monohydrochloride trihydrate from P-nitrobenzyl(1R,5S,6S)-2-diphenoxyphophoryloxy-6-[(R)-hydroxyethyl]-1-methyl-1-carbapen-2-em-3-carboxylic acid, which was derived from P-nitrobenzyl(1R,5S,6S)-2-diphenoxyphosphoryloxy-6-[(R)-1-hydroxyethyl]-1-methyl-1-carbapen-2-em-3-carboxylate ; reduction by means of 5% Pd/Al₂O₃ under a condition of two phase reaction 1-butanol/H₂O>

P-nitrobenzyl(1R,5S,6S)-2-diphenoxyphosphoryloxy-6-[(R)-1-hydroxyethyl]-1-methyl-1-carbapen-2-em-3-carboxylate (5.0 g, 8.41 mmol) was added into a 200 mL four-necked flask, and then, dimethylsulfoxide (45 mL) was added thereto. The mixture was dissolved at room temperature (20°C), and then, N-ethyldiisopropylamine (2.88 mL, 16.8 mmol, 2.0 eq.) was added thereto (at 17 to 18°C) with stirring. Then, (2S,4S)-2-[[(3R)-pyrrolidin-3-yl-(R)-hydroxy]methyl]-4-mercaptopyrrolidine dihydrochloride (2.55 g, 9.26 mmol, 1.1 eq.) powder was slowly added to the resulting mixture for five minutes with stirring. The admixture was maintained at 17 to 18°C for thirty minutes thereby allowing the same to react with each other.

The reaction solution was diluted with 1-butanol (60 mL). Separately, a mixed solution of distilled water (270 mL), 5% Pd/Al₂O₃ (6.0 g), and 1-butanol (60 mL) was previously prepared in a 500 mL four-necked flask equipped with a pH stat and a stirrer. To the mixed solution was added the resulting reaction solution with stirring. 1-Butanol (60 mL) was further added to the 200 mL flask to which addition of the reaction solution had been completed to wash the flask, and the washing liquids were also added to the 500 mL flask. During the addition of the reaction solution and the washing liquids, the temperature was maintained at 10°C or less, and the pH was maintained at 4.5 to 6.5. After the addition of the reaction solution and the washing liquids, the inside of the 500 mL flask was replaced with nitrogen. Thereafter, the admixture was vigorously stirred for four hours while adjusting a pH of the reaction solution to 6.0 by dropping 1N sodium hydroxide aqueous solution by means of a constant rate pump linked to the pH stat under hydrogen atmosphere (normal pressure, hydrogen feeding by means of a balloon).

The hydrogen feeding was stopped at the time when consumption of the 1N sodium hydroxide aqueous solution ceased (total 7.8 mL), and thereafter, a catalyst was filtered off by the use of a Kiriyama funnel (No. 5) . The remaining liquid was washed with distilled water (35 mL), and thereafter, the filtrate and the washing solutions were combined. After methanol (100 mL) was added to the solution of two layers, to be a homogeneous layer, a free type of the titled compound produced was subjected to HPLC determination. (3.06 g; total reaction yield from P-nitrobenzyl(1R,5S,6S)-2-diphenoxyphosphoryloxy-6-[(R)-1-hydroxyethyl]-1-methyl-1-carbapen-2-em-3-carboxylate : 88.4%)

### Example 6:

### <One-Pot reaction of (1R,5S,6S)-6-[(R)-1-hydroxyethyl]-1-methyl-2-{(2S,4S)-2-[(3R)-pyrrolidin-3-yl-(R)-hydroxy]methyl}pyrrolidine-4-ylthiol-1-carbapen-2-em-3-carboxylic acid monohydrochloride trihydrate from P-nitrobenzyl(1R,5S,6S)-2-diphenoxyphophoryloxy-6-[(R)-hydroxyethyl]-1-methyl-1-carbapen-2-em-3-carboxylic acid, which was derived from a starting material: P-nitrobenzyl(1R,5S,6S)-2-diphenoxyphosphoryloxy-6-[(R)-1-hydroxyethyl]-1-methyl-1-carbapen-2-em-3-carboxylate ; reduction by means of 5% Pt-C under a condition of two phase reaction 1-butanol/H₂O>

P-nitrobenzyl(1R,5S,65)-2-diphenoxyphosphoryloxy-6-[(R)-1-hydroxyethyl]-1-methyl-1-carbapen-2-em-3-carboxylate (5.0 g, 8.41 mmol) was added into a 200 mL four-necked flask, and then, dimethylsulfoxide (45 mL) was added thereto. The mixture was dissolved at room temperature (19°C) , and then, N-ethyldiisopropylamine (2.88 mL, 16.8 mmol, 2.0 eq.) was added thereto (at 17 to 18°C) with stirring. Then, (2S,4S)-2-[[(3R)-pyrrolidin-3-yl-(R)-hydroxy]methyl]-4-mercaptopyrrolidine dihydrochloride (2.55 g, 9.26 mmol, 1.1 eq.) powder was slowly added to the resulting mixture for five minutes with stirring. The admixture was maintained at 16 to 18°C for thirty minutes thereby allowing the same to react with each other.

The reaction solution was diluted with 1-butanol (60 mL). Separately, a mixed solution of distilled water (270 mL), 5% Pt-C (6.0 g), and 1-butanol (60 mL) was previously prepared in a 500 mL four-necked flask equipped with a pH stat and a stirrer. To the mixed solution was added the resulting reaction solution with stirring. 1-Butanol (60 mL) was further added to the 200 mL flask to which addition of the reaction solution had been completed to wash the flask, and the washing liquids were also added to the 500 mL flask. During the addition of the reaction solution and the washing liquids, the temperature was maintained at 10°C or less, and the pH was maintained at 4.5 to 6.5. After the addition of the reaction solution and the washing liquids, the inside of the 500 mL flask was replaced with nitrogen. Thereafter, the admixture was vigorously stirred for four hours while adjusting a pH of the reaction solution to 6.0 by dropping 1N sodium hydroxide aqueous solution by means of a constant rate pump linked to the pH stat under hydrogen atmosphere (normal pressure, hydrogen feeding by means of a balloon).

The hydrogen feeding was stopped at the time when consumption of the 1N sodium hydroxide aqueous solution ceased (total 9.6 mL), and thereafter, a catalyst was filtered off by the use of a Kiriyama funnel (No. 5). The remaining liquid was washed with distilled water (35 mL), and thereafter, the filtrate and the washing solutions were combined. After methanol (50 mL) was added to the solution of two layers, to be a homogeneous layer, a free type of the titled compound produced was subjected to HPLC determination. (2.61 g; total reaction yield from P-nitrobenzyl(1R,5S,6S)-2-diphenoxyphosphoryloxy-6-[(R)-1-hydroxyethyl]-1-methyl-1-carbapen-2-em-3-carboxylate : 75.6%)

### Example 7:

### <One-Pot reaction of (1R,5S,6S)-6-[(R)-1-hydroxyethyl]-1-methyl-2-[(2S,4S) 2 [(3R)-pyrrolidin-3-yl-(R)-hydroxy]methyl}pyrrolidine-4-ylthio]-1-carbapen-2-em-3-carboxylic acid monohydrochloride trihydrate from P-nitrobenzyl(1R,5S,6S)-2-diphenoxyphophoryloxy-6-[(R)-hydroxyethyl]-1-methyl-1-carbapen-2-em-3-carboxylic acid, which was derived from P-nitrobenzyl(1R,5S,6S)-2-diphenoxyphosphoryloxy-6-[(R)-1-hydroxyethyl]-1-methyl-1-carbapen-2-em-3-carboxylate ; reduction by means of 5% Pd-Black under a condition of two phase reaction 1-butanol/H₂O>

P-nitrobenzyl(1R,5S,6S)-2-diphenoxyphosphoryloxy-6-[(R)-1-hydroxyethyl]-1-methyl-1-carbapen-2-em-3-carboxylate (5.0 g, 8.41 mmol) was added into a 200 mL four-necked flask, and then, dimethylsulfoxide (45 mL) was added thereto. The mixture was dissolved at room temperature (24°C), and then, N-ethyldiisopropylamine (2.88 mL, 16.8 mmol, 2.0 eq.) was added thereto (at 22 to 23°C) with stirring. Then, (2S,4S)-2-[[(3R)-pyrrolidin-3-yl-(R)-hydroxy]methyl]-4-mercaptopyrrolidine dihydrochloride (2.55 g, 9.26 mmol, 1.1 eq.) powder was slowly added to the resulting mixture for five minutes with stirring. The admixture was maintained at 19 to 22°C for thirty minutes thereby allowing the same to react with each other.

The reaction solution was diluted with 1-butanol (60 mL). Separately, a mixed solution of distilled water (270 mL), 5% Pd-Black (5.0 g), and 1-butanol (60 mL) was previously prepared in a 500 mL four-necked flask equipped with a pH stat and a stirrer. To the mixed solution was added the resulting reaction solution with stirring. 1-Butanol (60 mL) was further added to the 200 mL flask to which addition of the reaction solution had been completed to wash the flask, and the washing liquids were also added to the 500 mL flask. During the addition of the reaction solution and the washing liquids, the temperature was maintained at 10°C or less, and the pH was maintained at 4.5 to 6.5. After the addition of the reaction solution and the washing liquids, the inside of the 500 mL flask was replaced with nitrogen. Thereafter, the admixture was vigorously stirred for three hours while adjusting a pH of the reaction solution to 6.0 by dropping 1N sodium hydroxide aqueous solution by means of a constant rate pump linked to the pH stat under hydrogen atmosphere (normal pressure, hydrogen feeding by means of a balloon).

The hydrogen feeding was stopped at the time when consumption of the 1N sodium hydroxide aqueous solution ceased (total 8.1 mL), and thereafter, a catalyst was filtered off by the use of a Kiriyama funnel (No. 5). The remaining liquid was washed with distilled water (35 mL), and thereafter, the filtrate and the washing solutions were combined. After methanol (100 mL) was added to the solution of two layers, to be a homogeneous layer, a free type of the titled compound produced was subjected to HPLC determination. (3.02 g; total reaction yield from P-nitrobenzyl(1R,5S,6S)-2-diphenoxyphosphoryloxy-6-[(R)-1-hydroxyethyl]-1-methyl-1-carbapen-2-em-3-carboxylate : 87.2%)

### Example 8:

### <One-Pot reaction of (1R,5S,6S)-6-[(R)-1-hydroxyethyl]-1-methyl-2-{(2S,4S)-2-[(3R)-pyrrolidin-3-yl-(R)-hydroxy]methyl}pyrrolindine-4-ylthio]-1-carbapen-2-em-3-carboxylic acid monohydrochloride trihydrate from P-nitrobenzyl(1R,5S,6S)-2-diphenoxyphophoryloxy-6-[(R)-hydroxyethyl]-1-methyl-1-carbapen-2-em-3-carboxylic acid, which was derived from P-nitrobenzyl(1R,5S,6S)-2-diphenoxyphosphoryloxy-6-[(R)-1-hydroxyethyl]-1-methyl-1-carbapen-2-em-3-carboxylate ; reduction by means of palladium oxide under a condition of two phase reaction 1-butanol/H₂O>

P-nitrobenzyl(1R,5S,6S)-2-diphenoxyphosphoryloxy-6-[(R)-1-hydroxyethyl]-1-methyl-1-carbapen-2-em-3-carboxylate (5.0 g, 8.41 mmol) was added into a 200 mL four-necked flask, and then, dimethylsulfoxide (45 mL) was added thereto. The mixture was dissolved at room temperature (21°C) , and then, N-ethyldiisopropylamine (2.88 mL, 16.8 mmol, 2.0 eq.) was added thereto (at 22°C) with stirring. Then, (2S,4S)-2-[[(3R)-pyrrolidin-3-yl-(R)-hydroxy]methyl]-4-mercaptopyrrolidine dihydrochloride (2.55 g, 9.26 mmol, 1.1 eq.) powder was slowly added to the resulting mixture for five minutes with stirring. The admixture was maintained at 20 to 22°C for thirty minutes thereby allowing the same to react with each other.

The reaction solution was diluted with 1-butanol (60 mL). Separately, a mixed solution of distilled water (270 mL), palladium oxide (0.6 g) and 1-butanol (60 mL) was previously prepared in a 500 mL four-necked flask equipped with a pH stat and a stirrer. To the mixed solution was added the resulting reaction solution with stirring. 1-Butanol (60 mL) was further added to the 200 mL flask to which addition of the reaction solution had been completed to wash the flask, and the washing liquids were also added to the 500 mL flask. During the addition of the reaction solution and the washing liquids, the temperature was maintained at 10°C or less, and the pH was maintained at 4.5 to 6.5. After the addition of the reaction solution and the washing liquids, the inside of the 500 mL flask was replaced with nitrogen. Thereafter, the admixture was vigorously stirred for two hours while adjusting a pH of the reaction solution to 6.0 by dropping 1N sodium hydroxide aqueous solution by means of a constant rate pump linked to the pH stat under hydrogen atmosphere (normal pressure, hydrogen feeding by means of a balloon).

The hydrogen feeding was stopped at the time when consumption of the 1N sodium hydroxide aqueous solution ceased (total 8.5 mL), and thereafter, a catalyst was filtered off by the use of a Kiriyama funnel (No. 5). The remaining liquid was washed with distilled water (35 mL) , and thereafter, the filtrate and the washing solutions were combined. After methanol (100 mL) was added to the solution of two layers, to be a homogeneous layer, a free type of the titled compound produced was subjected to HPLC determination. (3.00 g; total reaction yield from P-nitrobenzyl(1R,5S,6S)-2-diphenoxyphosphoryloxy-6-[(R)-1-hydroxyethyl]-1-methyl-1-carbapen-2-em-3-carboxylate : 86.9%)

### Example 9:

### <One-Pot reaction of (1R,5S,6S)-6-[(R)-1-hydroxyethyl)-1-methyl-2-((2S,4S)-2-[(3R)-pyrrolidin-3-yl-(R)-hydroxy]methyl}pyrrolidine-4-ylthio]-1-carbapen-2-em-3-carboxylic acid monohydrochloride trihydrate from P-nitrobenzyl(1R,5S,6S)-2-diphenoxyphophoryloxy-6-[(R)-hydroxyethyl]-1-methyl-1-carbapen-2-em-3-carboxylic acid, which was derived from P-nitrobenzyl(1R,5S,6S)-2-diphenoxyphosphoryloxy-6-[(R)-1-hydroxyethyl]-1-methyl-1-carbapen-2-em-3-carboxylate ; reduction by means of 5% Pd/BaSO₄ under a condition of two phase reaction 1-butanol/H₂O>

P-nitrobenzyl(1R,5S,6S)-2-diphenoxyphosphoryloxy-6-[(R)-1-hydroxyethyl]-1-methyl-1-carbapen-2-em-3-carboxylate (5.0 g, 8.41 mmol) was added into a 200 mL four-necked flask, and then, a mixed solution of dimethylsulfoxide (18 mL) and dimethylformamide (4.5 mL) was added thereto. The mixture was dissolved at room temperature (12°C), and then, N-ethyldiisopropylamine (2.88 mL, 16.8 mmol, 2.0 eq.) was added (at 11°C) with stirring. Then, (2S,4S)-2-[[(3R)-pyrrolidin-3-yl-(R)-hydroxy]methyl]-4-mercaptopyrrolidine dihydrochloride (2.55 g, 9.26 mmol, 1.1 eq.) powder was slowly added to the resulting mixture for five minutes with stirring. The admixture was maintained at 8 to 10°C for one hour thereby allowing the same to react with each other.

The reaction solution was diluted with 1-butanol (60 mL). Separately, a mixed solution of distilled water (135 mL), Pd/BaSO₄ (20.0 g), and 1-butanol (30 mL) was previously prepared in a 500 mL four-necked flask equipped with a pH stat and a stirrer. To the mixed solution was added the resulting reaction solution with stirring. 1-Butanol (30 mL) was further added to the 200 mL flask to which addition of the reaction solution had been completed to wash the flask, and the washing liquids were also added to the 500 mL flask. During the addition of the reaction solution and the washing liquids, the temperature was maintained at 10°C or less, and the pH was maintained at 4.5 to 6.5. After the addition of the reaction solution and the washing liquids, the inside of the 500 mL flask was replaced with nitrogen. Thereafter, the admixture was vigorously stirred for two hours while adjusting a pH of the reaction solution to 6.0 by dropping 1N sodium hydroxide aqueous solution by means of a constant rate pump linked to the pH stat under hydrogen atmosphere (normal pressure, hydrogen feeding by means of a balloon).

The hydrogen feeding was stopped at the time when consumption of the 1N sodium hydroxide aqueous solution ceased (total 7. 6 mL), and thereafter, a catalyst was filtered off by the use of a Kiriyama funnel (No. 5) . The remaining liquid was washed with distilled water (35 mL), and thereafter, the filtrate and the washing solutions were combined. After methanol (100 mL) was added to the solution of two layers, to be a homogeneous layer, a free type of the titled compound produced was subjected to HPLC determination. (2.99 g; total reaction yield from P-nitrobenzyl(1R,5S,6S)-2-diphenoxyphosphoryloxy-6-[(R)-1-hydroxyethyl]-1-methyl-1-carbapen-2-em-3-carboxylate : 86.4%)

### Example 10:

### <One-Pot reaction of (1R,5S,6S)-6-[(R)-1-hydroxyethyl]-1-methyl-2-{(2S,4S)-2-[(3R)-pyrrolidin-3-yl-(R)-hydroxy]methyl}pyrrolidine-4-ylthio]-1-carbapen-2-em-3-carboxylic acid monohydrochloride trihydrate from P-nitrobenzyl(1R,5S,6S)-2-diphenoxyphophoryloxy-6-[(R)-hydroxyethyl]-1-methyl-1-carbapen-2-em-3-carboxylic acid, which was derived from P-nitrobenzyl(1R,5S,6S)-2-diphenoxyphosphoryloxy-6-[(R)-1-hydroxyethyl]-1-methyl-1-carbapen-2-em-3-carboxylate ; reduction by means of 5% Ir-C under a condition of two phase reaction 1-butanol/H₂O>

P-nitrobenzyl(1R,5S,6S)-2-diphenoxyphosphoryloxy-6-[(R)-1-hydroxyethyl]-1-methyl-1-carbapen-2-em-3-carboxylate (5.0 g, 8.41 mmol) was added into a 200 mL four-necked flask, and then, dimethylsulfoxide (45 mL) was added thereto. The mixture was dissolved at room temperature (20°C), and then, N-ethyldiisopropylamine (2.88 mL, 16.8 mmol, 2.0 eq.) was added thereto (at 17°C) with stirring. Then, (2S,4S)-2-[[(3R)-pyrrolidin-3-yl-(R)-hydroxy]methyl]-4-mercaptopyrrolidine dihydrochloride (2.55 g, 9.26 mmol, 1.1 eq.) powder was slowly added to the resulting mixture for five minutes with stirring. The admixture was maintained at 18 to 20°C for thirty minutes thereby allowing the same to react with each other.

The reaction solution was diluted with 1-butanol (60 mL) . Separately, a mixed solution of distilled water (270 mL), 5% Pd/BaSO₄ (20.0 g), and 1-butanol (30 mL) was previously prepared in a 500 mL four-necked flask equipped with a pH stat and a stirrer. To the mixed solution was added the resulting reaction solution with stirring. 1-Butanol (30 mL) was further added to the 200 mL flask to which addition of the reaction solution had been completed to wash the flask, and the washing liquids were also added to the 500 mL flask. During the addition of the reaction solution and the washing liquids, the temperature was maintained at 10°C or less, and the pH was maintained at 4.5 to 6.5. After the addition of the reaction solution and the washing liquids, the inside of the 500 mL flask was replaced with nitrogen. Thereafter, the admixture was vigorously stirred for two hours while adjusting a pH of the reaction solution to 6.0 by dropping 1N sodium hydroxide aqueous solution by means of a constant rate pump linked to the pH stat under hydrogen atmosphere (normal pressure, hydrogen feeding by means of a balloon). After two hours, consumption of the 1N sodium hydroxide aqueous solution was 0.8 mL, just to collect starting materials.

### Example 11:

### <One-Pot reaction of (1R,5S,6S)-6-[(R)-1-hydroxyethyl]-1-methyl-2-{(2S,4S)-2-[(3R)-pyrrolidin-3-yl-(R)-hydroxy]methyl}pyrrolidine-4-ylthio]-1-carbapen-2-em-3-carboxylic acid monohydrochloride trihydrate from P-nitrobenzyl(1R,5S,6S)-2-diphenoxyphophoryloxy-6-[(R)-hydroxyethyl]-1-methyl-1-carbapen-2-em-3-carboxylic acid, which was derived from P-nitrobenzyl(1R,5S,6S)-2-diphenoxyphosphoryloxy-6-[(R)-1-hydroxyethyl]-1-methyl-1-carbapen-2-em-3-carboxylate ; reduction by means of 5% Pd/Al₂O₃ under a condition of two phase reaction 1-butanol/H₂O (without water concentration)>

P-nitrobenzyl(1R,5S,6S)-2-diphenoxyphosphoryloxy-6-[(R)-1-hydroxyethyl]-1-methyl-1-carbapen-2-em-3-carboxylate (10.0 g, 16.82 mmol) was added into a 200 mL four-necked flask, and then, dimethylsulfoxide (45 mL) was added thereto. The mixture was dissolved at room temperature (16°C), and then, N-ethyldiisopropylamine (7.32 mL, 42.7 mmol, 2.0 eq.) was added thereto (at 16°C) with stirring. Then, (2S,4S)-2-[[(3R)-pyrrolidin-3-yl-(R)-hydroxy]methyl]-4-mercaptopyrrolidine monohydrochloride (5.10 g, 18.52 mmol, 1.1 eq.) powder was slowly added to the resulting mixture for ten minutes with stirring. The admixture was maintained at 16 to 18°C for fourty five minutes thereby allowing the same to react with each other.

The reaction solution was diluted with 1-butanol (15 mL) . Separately, a mixed solution of distilled water (135 mL), 5% Pd/Al₂O₃ (24.0 g) and 1-butanol (30 mL) was previously prepared in a 500 mL four-necked flask equipped with a pH stat and a stirrer. To the mixed solution was added the resulting reaction solution with stirring. 1-Butanol (15 mL) was further added to the 200 mL flask to which addition of the reaction solution had been completed to wash the flask, and the washing liquids were also added to the 500 mL flask. During the addition of the reaction solution and the washing liquids, the temperature was maintained at 10°C or less, and the pH was maintained at 4.5 to 6.5. After the addition of the reaction solution and the washing liquids, the inside of the 500 mL flask was replaced with nitrogen. Thereafter, the admixture was vigorously stirred for two hours and half while adjusting a pH of the reaction solution to 6.0 by dropping 1N sodium hydroxide aqueous solution by means of a constant rate pump linked to the pH stat under hydrogen atmosphere (normal pressure, hydrogen feeding by means of a balloon).

The hydrogen feeding was stopped at the time when consumption of the 1N sodium hydroxide aqueous solution ceased (total 12.7 mL), and thereafter, a catalyst was filtered off by the use of a Kiriyama funnel (No. 5) . The remaining liquid was washed with distilled water (15 mL), and thereafter, the filtrate and the washing solutions were combined. When the organic layer and the water layer were subjected to HPLC determination, respectively, it was found that a free type of the titled compound produced was as follows. (Water layer: 5.64 g; organic layer: 0.100 g; total reaction yield from P-nitrobenzyl(1R,5S,6S)-2-diphenoxyphosphoryloxy-6-[(R)-1-hydroxyethyl]-1-methyl-1-carbapen-2-em-3-carboxylate : 82.9%)

The above-described water layer was treated with activated carbon (Taikoh FCS, 1.0 g) for 10 minutes, while the water layer was cooled in an ice-bath, and then, the activated carbon was filtered off. A filtrate (250 g) combined with wash liquid (10 mL) for the filter residue was cooled to 10°C. To the resulting solution, IPA (350 mL) was added dropwise with stirring. After one hour, precipitate of crystal was confirmed, and 2-propanol (400 mL) was added dropwise thereto. The admixture was crystallized at 10°C for 14 hours, thereafter, the crystal was filtered out by means of suction filtration. The crystal was washed with 80% cooled 2-propanol aqueous solution (60 mL), 2-propanol (60 mL), and acetone (60 mL) in this order. The resulting crystal was dried by means of suction for two hours under flowing nitrogen, to obtain 6.20 g of the titled compound (corresponding to 5.25 g when converted into the free type; total yield from P-nitrobenzyl(1R,5S,6S)-2-diphenoxyphosphoryloxy-6-[(R)-1-hydroxyethyl]-1-methyl-1-carbapen-2-em-3-carboxylate : 75.9%).

After dissolving 2.01 g (corresponding to 1.70 g in the form of the free type thereof) of (1R,5S,6S)-6-[(R)-1-hydroxylethyl]-1-methyl-2-{(2S,4S)-2-[(3R)-pyrrolidin-3-yl-(R)-hydroxy]methyl}pyrrolidin-4-ylthio]-1-carbapen-2-em-3-carboxylic acid monohydrochloride trihydrate into distilled water (14 mL), contaminant was filtered off (0.2 µm filter) from the resulting solution. 2-Propanol (20 mL) was added dropwise to an aqueous solution combined with washing aqueous solution (6 mL) with stirring the mixture under cooling at 10°C. Precipitate of crystal was confirmed. After aging for two hours, the crystal precipitated was collected, followed by washing with 80% (v/v) cooled 2-propanol aqueous solution (20 mL), 2-propanol (20 mL), and acetone (20 mL). The product was dried (for one hour) on the filter while depressurizing and drafting the same under nitrogen stream to obtain 1.90 g of the target crystal of the titled compound (corresponding to 1.58 g when converted into the free type; recovery: 92.8%).

### Example 12:

### <One-Pot reaction of (1R,5S,6S)-6-[(R)-1-hydroxyethyl]-1-methyl-2-{(2S,4S)-2-[(3R)-pyrrolidin-3-yl-(R)-hydroxy]methyl}pyrrolidine-4-ylthio]-1-carbapen-2-em-3-carboxylic acid monohydrochloride trihydrate from P-nitrobenzyl(1R,5S,6S)-2-diphenoxyphophoryloxy-6-[(R)-hydroxyethyl]-1-methyl-1-carbapen-2-em-3-carboxylic acid, which was derived from P-nitrobenzyl(1R,5S,6S)-2-diphenoxyphosphoryloxy-6-[(R)-1-hydroxyethyl]-1-methyl-1-carbapen-2-em-3-carboxylate ; reduction by means of 5% Pd/Al₂O₃ under a condition of two phase reaction 3-methyl-1-butanol/H₂O (without water concentration) >

P-nitrobenzyl(1R,5S,6S)-2-diphenoxyphosphoryloxy-6-[(R)-1-hydroxyethyl]-1-methyl-1-carbapen-2-em-3-carboxylate (10.0 g, 16.82 mmol) was added into a 200 mL four-necked flask, and then, dimethylsulfoxide (90 mL) was added thereto. The mixture was dissolved at room temperature (17°C), and then, N-ethyldiisopropylamine (7.32 mL, 42. 7 mmol, 2.0 eq.) was added thereto (at 16°C) with stirring. Then, (2S,4S)-2-[[(3R)-pyrrolidin-3-yl-(R)-hydroxy]methyl]-4-mercaptopyrrolidine monohydrochloride (5.10 g, 18.52 mmol, 1.1 eq.) powder was slowly added to the resulting mixture for seventeen minutes with stirring. The admixture was maintained at 16 to 18°C for forty-three minutes thereby allowing the same to react with each other.

The reaction solution was diluted with 3-methyl-1-butanol (60 mL). Separately, a mixed solution of distilled water (270 mL), 5% Pd/Al₂O₃ (18.0 g) and 3-methyl-1-butanol (100 mL) was previously prepared in a 500 mL four-necked flask equipped with a pH stat and a stirrer. To the mixed solution was added the resulting reaction solution with stirring. 3-Methyl-1-butanol (20 mL) was further added to the 200 mL flask to which addition of the reaction solution had been completed to wash the flask, and the washing liquids were also added to the 500 mL flask. During the addition of the reaction solution and the washing liquids, the temperature was maintained at 10°C or less, and the pH was maintained at 4.5 to 6.5. After the addition of the reaction solution and the washing liquids, the inside of the 500 mL flask was replaced with nitrogen. Thereafter, the admixture was vigorously stirred for two hours while adjusting a pH of the reaction solution to 6.0 by dropping 1N sodium hydroxide aqueous solution by means of a constant rate pump linked to the pH stat under hydrogen atmosphere (normal pressure, hydrogen feeding by means of a balloon).

The hydrogen feeding was stopped at the time when consumption of the 1N sodium hydroxide aqueous solution ceased (total 13.1 mL), and thereafter, a catalyst was filtered off by the use of a Kiriyama funnel (No. 5) . Water layer was washed with 3-methyl-1-butanol (80 mL), and then was combined with organic layer. When the organic layer and the water layer were subjected to HPLC determination, respectively, it was found that a free type of the titled compound produced was as follows. (Water layer: 5.90 g; organic layer: 0.154 g; total reaction yield from P-nitrobenzyl (1R, 5S, 6S)-2-diphenoxyphosphoryloxy-6-[(R)-1-hydroxyethyl]-1-methyl-1-carbapen-2-em-3-carboxylate : 87.5%)

The above-described water layer was treated with activated carbon (Taikoh FCS, 1.0 g) for 10 minutes, while the water layer was cooled in an ice-bath, and then, the activated carbon was filtered off. A filtrate (411 g) combined with wash liquid (10 mL) for the filter residue was cooled to 10°C. To the resulting solution, 2-propanol (520 mL) was added dropwise with stirring. After two hours, precipitate of crystal was confirmed, and dimethylsulfoxide (230 mL) was added dropwise thereto. After one hour, 2-propanol (220 mL) was added dropwise thereto. The admixture was crystallized at 10°C for 14 hours, thereafter, the crystal was filtered out by means of suction filtration. The crystal was washed with 80% cooled 2-propanol aqueous solution (80 mL), 2-propanol (80 mL), and acetone (80 mL) in this order. The resulting crystal was dried by means of suction for two hours under flowing nitrogen, to obtain 6.37 g of the titled compound (corresponding to 5.45 g when converted into the free type; total yield from P-nitrobenzyl(1R,5S,6S)-2-diphenoxyphosphoryloxy-6-[(R)-1-hydroxyethyl]-1-methyl-1-carbapen-2-em-3-carboxylate : 78.7%).

After dissolving 2.00 g (corresponding to 1.71 g in the form of the free type thereof) of (1R,5S,6S)-6-[(R)-1-hydroxylethyl]-1-methyl-2-{(2S,4S)-2-[(3R)-pyrrolidin-3-yl-(R)-hydroxy]methyl}pyrrolidin-4-ylthio]-1-carbapen-2-em-3-carboxylic acid monohydrochloride trihydrate into distilled water (14 mL), contaminant was filtered off (0.2 µm filter) from the resulting solution. 2-Propanol (20 mL) was added dropwise to an aqueous solution combined with washing aqueous solution (6 mL) with stirring the mixture under cooling at 10°C. Precipitate of crystal was confirmed. After aging for one hour, further 2-propanol (60 mL) was added thereto for one hour. After aging for two hours, the crystal precipitated was collected, followed by washing with 80% (v/v) cooled 2-propanol aqueous solution (20 mL), 2-propanol (20 mL), and acetone (20 mL). The product was dried (for one hour) on the filter while depressurizing and drafting the same under nitrogen stream to obtain 1.91 g of the target crystal of the titled compound (corresponding to 1.60 g when converted into the free type; recovery: 93.6%; total yield from P-nitrobenzyl(1R,5S,6S)-2-diphenoxyphosphoryloxy-6-[(R)-1-hydroxyethyl]-1-methyl-1-carbapen-2-em-3-carboxylate : 73.7%).

## Claims

1. A method for producing a compound represented by the general formula IV or the salt thereof, or the hydrates thereof, comprising the steps of:
reacting a compound represented by the following general formula I (wherein R¹ represents a hydrogen atom or a methyl group, R² represents a hydrogen atom or a protective group for hydroxyl group, and R³ represents a hydrogen atom or a protective group for carboxyl group) with a compound represented by X-H (the general formula II) (wherein X represents anyone of substituents selected from the group consisting of the following formulae X-1 through X-8) to obtain a compound represented by the following general formula III (wherein R¹, R², R³, and X have the same meanings as defined above) or the salt thereof;
deprotecting the protective group R³ to prepare a compound represented by the general formula IV (wherein R¹, R², and X have the same meanings as defined above) or the salt thereof; and
crystallizing the compound represented by the general formula IV or the salt thereof thereby to purify the product:

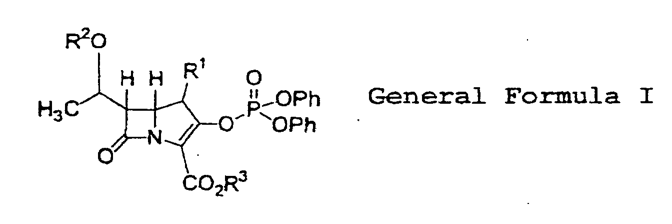

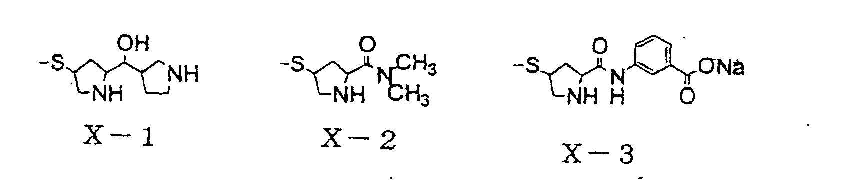

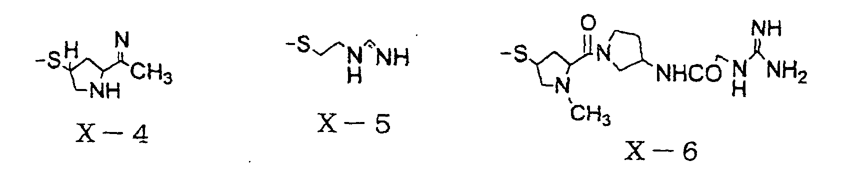

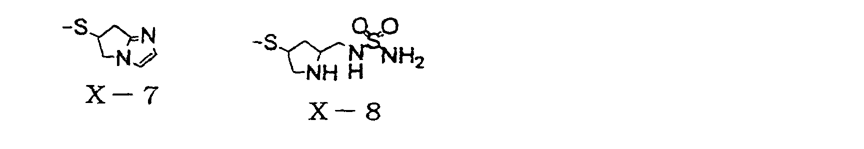

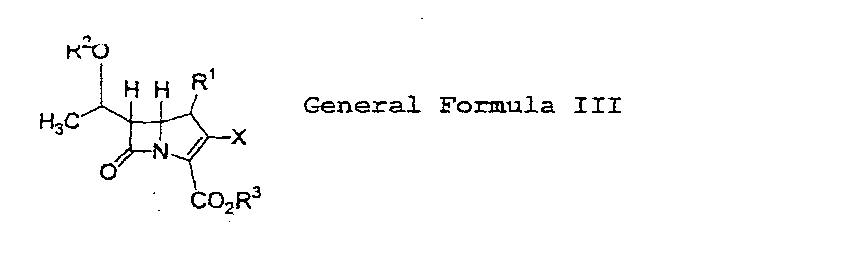

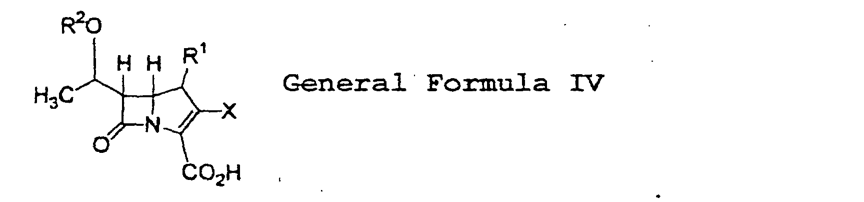

2. A method for producing a compound represented by the general formula IV or the salt thereof, or the hydrates thereof, comprising the step of:
crystallizing a compound represented by the following general formula IV (wherein R¹ represents a hydrogen atom or a methyl group, R² represents a hydrogen atom or a protective group for hydroxyl group, and X represents anyone of substituents selected from the group consisting of the following formulae X-1 through X-8), thereby to purify the product:

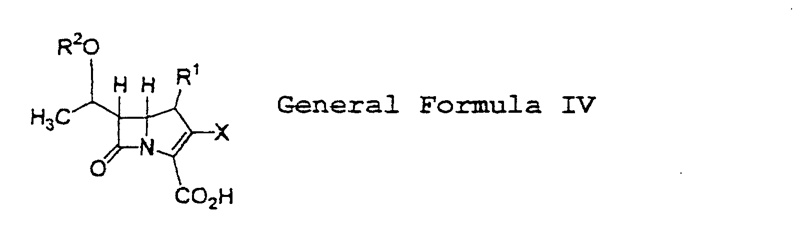

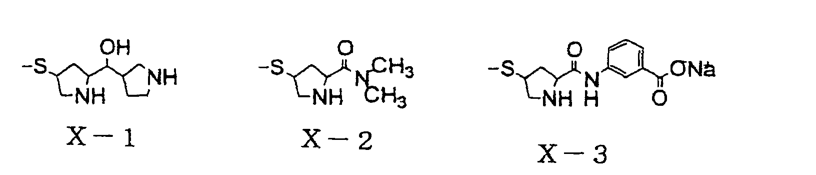

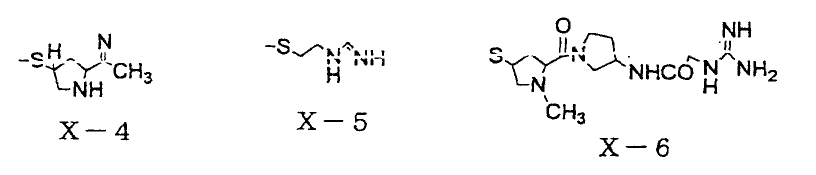

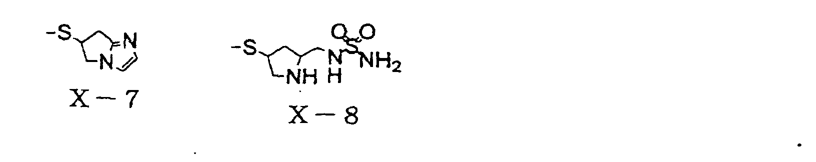

3. The method according to claim 1, wherein the production is carried out in accordance with one pot manner without an isolating step.

4. The method according to any one of claims 1 to 3, wherein X is a substituent selected from the group consisting of formulae X-1 through X-6:

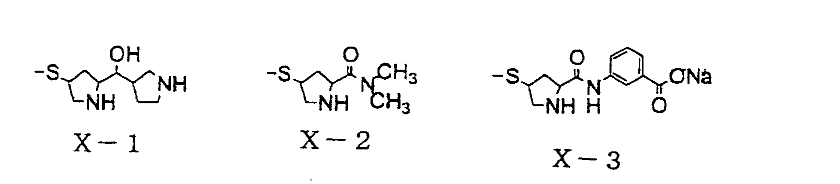

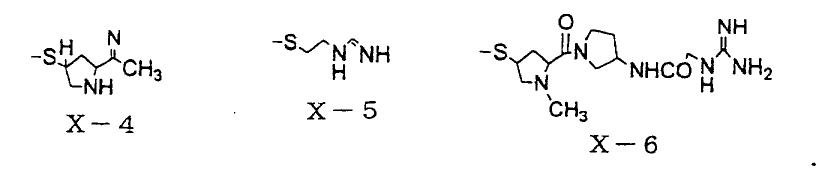

5. The method according to any one of claims 1 to 3, wherein X is the formula X-1:

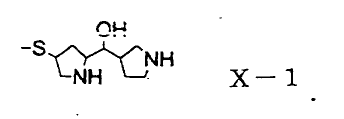

6. The method according to any one of claims 1, and 3 to 5, wherein said deprotecting step is carried out by catalytic reduction.

7. The method according to any one of claims 1 to 6, wherein said crystallizing step is carried out with the use of a solvent containing at least one member selected from the group consisting of alcohol-base solvents, dimethylsulfoxide, and dimethylformamide.

8. The method according to claim 7, wherein said alcohol-base solvent is at least one member selected from the group consisting of methanol, ethanol, 1-propanol, 2-propanol, 1-butanol, 2-butanol, 2-methyl-1-propanol, 2-methyl-2-propanol, 1-pentanol, 2-pentanol, and 3-pentanol.

9. A method for producing a compound represented by the general formula IV' or the salt thereof, or the hydrates thereof, comprising the steps of:
reacting a compound represented by the following general formula I' (wherein R¹ represents a hydrogen atom or a methyl group, R² represents a hydrogen atom or a protective group for hydroxyl group, and R³ represents a hydrogen atom or a protective group for carboxyl group) with a compound represented by X'-H (the general formula II') (wherein X' represents anyone of substituents selected from the group consisting of the following formulae X'-1 through X'-8) to obtain a compound represented by the following general formula III' (wherein R¹, R², R³, and X' have the same meanings as defined above) or the salt thereof;
deprotecting the protective group R³ to prepare a compound represented by the general formula IV' (wherein R¹, R², and X' have the same meanings as defined above) or the salt thereof; and
crystallizing the compound represented by the general formula IV' or the salt thereof thereby to purify the product:

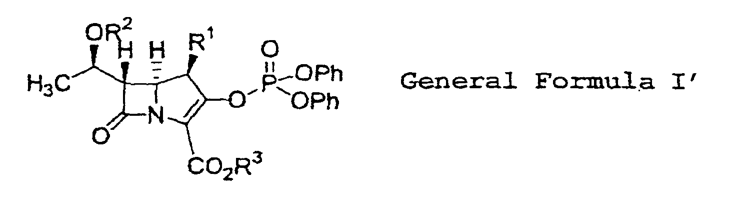

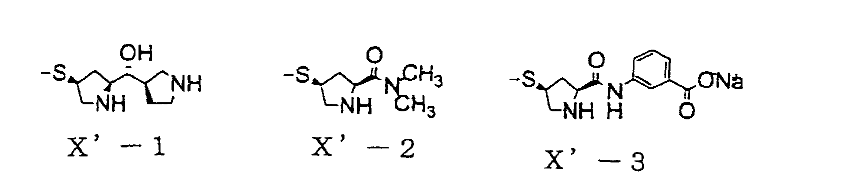

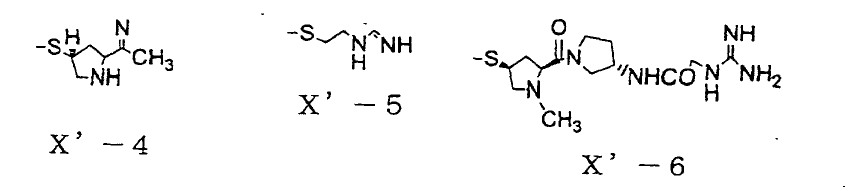

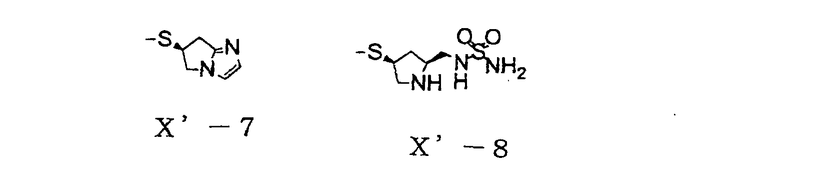

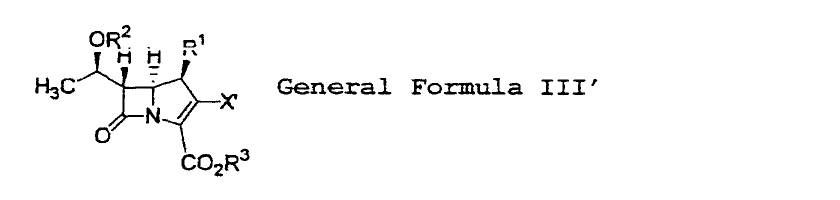

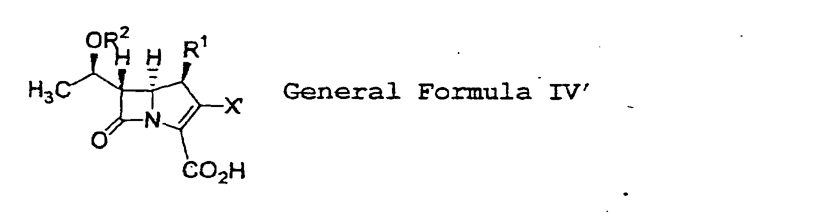

10. A method for producing a compound represented by the general formula IV' or the salt thereof, or the hydrates thereof, comprising the step of:
crystallizing a compound represented by the following general formula IV' (wherein R¹ represents a hydrogen atom or a methyl group, R² represents a hydrogen atom or a protective group for hydroxyl group, and X' represents anyone of substituents selected from the group consisting of the following formulae X'-1 through X'-8), thereby to purify the product:

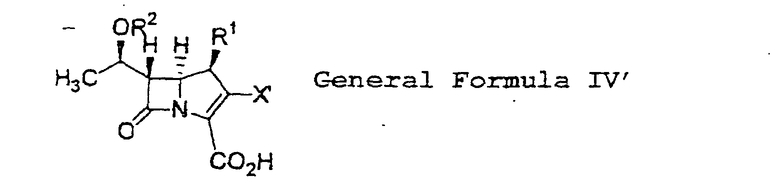

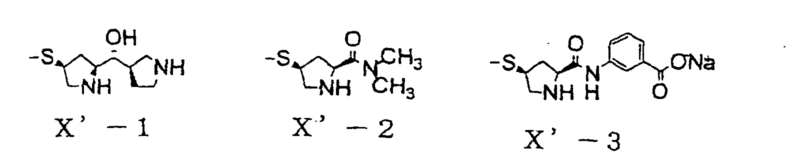

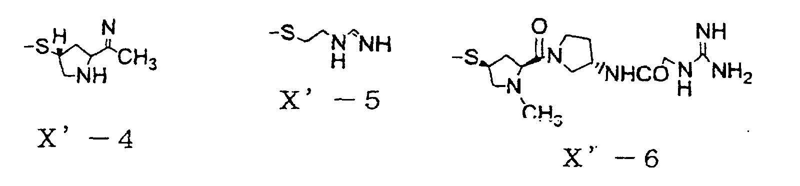

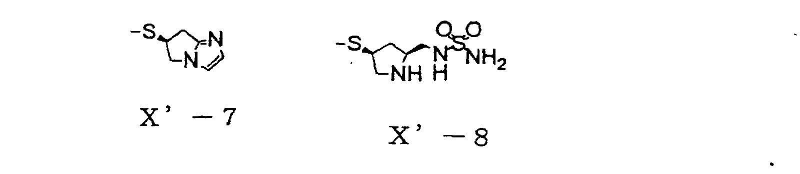

11. The method according to claim 9, wherein the production is carried out in accordance with one pot manner without an isolating step.

12. The method according to any one of claims 9 to 11, wherein X' is a substituent selected from the group consisting of formulae X'-1 through X'-6:

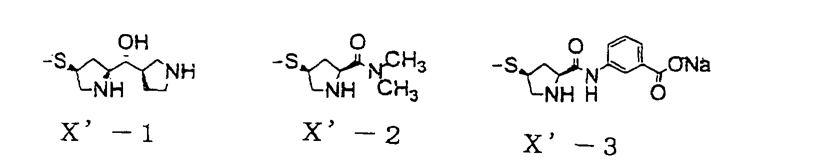

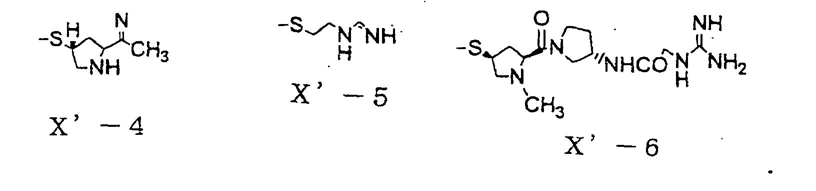

13. The method according to any one of claims 9 to 11, wherein X' is the formula X'-1:

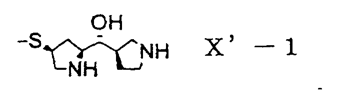

14. The method according to any one of claims 9 and 11 to 13, wherein said deprotecting step is carried out by catalytic reduction.

15. The method according to any one of claims 9 to 14, wherein said crystallizing step is carried out with the use of a solvent containing at least one member selected from the group consisting of alcohol-base solvents, dimethylsulfoxide, and dimethylformamide.

16. The method according to claim 15, wherein said alcohol-base solvent is at least one member selected from the group consisting of methanol, ethanol, 1-propanol, 2-propanol, 1-butanol, 2-butanol, 2-methyl-1-propanol 2-methyl-2-propanol, 1-pentanol, 2-pentanol, and 3-pentanol.

17. The method according to any of claims 1, 3 to 9, and 10 to 16, wherein a reaction of the compound represented by the general formula I with the compound represented by the general formula II, or a reaction of the compound represented by the general formula I' with the compound represented by the general formula II' is carried out with a use of a solvent containing dimethylsulfoxide and/or dimethylformamide.

18. The method according to any of claims 1, 3 to 9, and 10 to 17, wherein a protective group for the compound represented by the general formula III or III' is deprotected with the use of a solvent containing dimethylsulfoxide and/or dimethylformamide to obtain the compound represented by the general formula IV or IV'.
